# FASCICULE DE BREVET EUROPEEN

(11) **EP 2 168 633 B1**
(45) Date de publication et mention de la délivrance du brevet: **30.03.2016**
(21) Numéro de dépôt: 09305896.4
(22) Date de dépôt: 24.09.2009
(51) Int. Cl.: A61Q 5/06, A61K 8/58, A61K 8/891, A61K 8/892, A61K 8/898, A61Q 5/00

(54) **Composition cosmétique comprenant un composé organique du silicium comportant au moins une fonction basique, un polymère filmogène hydrophobe, un pigment et un solvant volatil**
Kosmetischen Zusammensetzung enthaltend organische Silicium-Derivate mit einer basischen Gruppe als Vorprodukt vor einer Zusammensetzung enthaltend ein filmbildneres hydrophobisches Polymer, ein Pigment und ein Lösungsmittel
Cosmetic composition comprising organic derivatives of silicium containing at least a basic moiety as pre-treatment before a composition comprising a film-forming hydrophobic polymer, a pigment and a solvent

(30) Priorité: 30.09.2008 FR 0856597; 30.09.2008 FR 0856598
(43) Date de publication de la demande: 31.03.2010
(73) Titulaire: L'Oréal, 75008 Paris (FR)
(72) Inventeur: Brun, Gaëlle, 75011, Paris (FR); Bonnamy, Arnaud, 78000, Versailles (FR)
(74) Mandataire: Dossmann, Gérard

(56) Documents cités:
- EP-A- 0 159 628
- EP-A- 1 767 187
- EP-A- 1 767 189
- WO-A-01/22925
- WO-A-97/18795
- WO-A1-98/44906
- WO-A2-01/74308
- FR-A- 2 907 678
- US-A- 4 344 763
- US-A- 5 281 240
- US-A1- 2006 110 351

## Description

La présente invention concerne une composition cosmétique prête à l'emploi pour le traitement des fibres kératiniques, en particulier des fibres kératiniques humaines telles que les cheveux, comprenant un ou plusieurs composés organiques du silicium convenablement sélectionnés, un ou plusieurs polymères filmogènes hydrophobes, un ou plusieurs pigments et un ou plusieurs solvants volatils. Elle concerne également un procédé de traitement cosmétique des fibres kératiniques ainsi qu'une utilisation mettant en oeuvre ladite composition.

La présente invention a de même pour objet l'utilisation d'une composition cosmétique comprenant, dans un milieu cosmétiquement acceptable, un ou plusieurs composés organiques du silicium tels que décrits ci-avant en pré-traitement d'une composition comprenant un ou plusieurs polymères filmogènes hydrophobes, un ou plusieurs pigments et un ou plusieurs solvants volatils.

Les cheveux sont généralement abîmés et fragilisés par l'action des agents atmosphériques extérieurs tels que la lumière et les intempéries, et par des traitements mécaniques ou chimiques tels que le brossage, le peignage, les décolorations, les permanentes et / ou les teintures. Il en résulte que les cheveux sont souvent difficiles à discipliner, en particulier ils sont difficiles à démêler ou à coiffer, et les chevelures, même abondantes, conservent difficilement une coiffure de bon aspect en raison du fait que les cheveux manquent de vigueur, de volume et de nervosité.

Cette dégradation des cheveux est par ailleurs accrue par la répétition de traitements de coloration permanente des cheveux, qui consiste à appliquer sur les cheveux un ou plusieurs précurseurs de colorant et un agent oxydant.

Ainsi, pour remédier à cela, il est maintenant usuel d'appliquer des produits de coiffage qui permettent de conditionner les cheveux en leur apportant notamment du corps, de la masse ou du volume.

Ces produits de coiffage sont généralement des compositions cosmétiques capillaires comprenant un ou plusieurs polymères qui présentent une forte affinité pour les cheveux et qui ont le plus souvent pour fonction de former un film à leur surface en vue de modifier leurs propriétés superficielles, notamment pour les conditionner ou pour leur apporter des propriétés optiques particulières.

Un inconvénient lié à l'utilisation de ces compositions capillaires réside dans le fait que les effets cosmétiques conférés par de telles compositions ont tendance à disparaître, notamment dès le premier shampooing.

Par ailleurs, dans le domaine de la coloration des fibres kératiniques, il est déjà connu de colorer des fibres kératiniques par différentes techniques à partir de colorants directs pour des colorations non permanentes ou de précurseurs de colorant pour des colorations permanentes.

La coloration non permanente ou coloration directe consiste à teindre les fibres kératiniques avec des compositions tinctoriales contenant des colorants directs. Ces colorants sont des molécules colorées et colorantes ayant une affinité pour les fibres kératiniques. Ils sont appliqués sur les fibres kératiniques pendant un temps nécessaire à l'obtention de la coloration désirée, puis rincés.

Les colorants classiques qui sont utilisés sont en particulier des colorants du type nitré benzénique, anthraquinonique, nitropyridinique, azoïque, xanthénique, acridinique, azinique, triarylméthane ou des colorants naturels.

Certains de ces colorants peuvent être utilisés dans des conditions éclaircissantes ce qui permet d'obtenir des colorations visibles sur des cheveux foncés.

Il est aussi connu de teindre les fibres kératiniques de façon permanente par la coloration d'oxydation. Cette technique de coloration consiste à appliquer sur les fibres kératiniques une composition contenant des précurseurs de colorant tels que des bases d'oxydation et des coupleurs. Ces précurseurs sous l'action d'un agent oxydant vont former dans le cheveu une ou plusieurs espèces colorées.

La variété des molécules mises en jeu au niveau des bases d'oxydation et des coupleurs, permet l'obtention d'une riche palette de couleurs et les colorations qui en résultent sont généralement puissantes, et résistantes aux agents extérieurs, notamment à la lumière, aux intempéries, aux lavages, à la transpiration et aux frottements.

Pour être visible sur cheveux foncés, ces deux techniques de coloration nécessitent une décoloration préalable ou simultanée des fibres kératiniques. Cette étape de décoloration mise en oeuvre avec un agent oxydant tel que le peroxyde d'hydrogène ou de persels entraîne une dégradation non négligeable des fibres kératiniques ce qui altère leurs propriétés cosmétiques. Les cheveux ont alors tendance à devenir rêches, plus difficilement démêlables et plus fragiles.

Une autre méthode de coloration consiste à utiliser des pigments. En effet, l'utilisation de pigments à la surface des fibres kératiniques permet en général d'obtenir des colorations visibles sur cheveux foncés puisque le pigment en surface masque la couleur naturelle de la fibre. L'utilisation de pigment pour colorer des fibres kératiniques est par exemple décrite dans la demande de brevet FR 2 741 530, qui préconise l'utilisation pour la coloration temporaire des fibres kératiniques d'une composition comprenant au moins une dispersion de particules de polymère filmogène comportant au moins une fonction acide et au moins un pigment dispersé dans la phase continue de ladite dispersion.

Les colorations obtenues par ce mode de coloration présentent l'inconvénient de s'éliminer dès le premier shampooing.

Pour remédier à ces inconvénients, il a été proposé d'effectuer des gainages en appliquant sur les cheveux une composition comprenant un polymère filmogène hydrophobe, en particulier un copolymère bloc polysiloxane / polyurée comme dans la demande de brevet FR 2 907 677. Une telle composition permet d'obtenir des cheveux parfaitement gainés et non gras. Les gainages obtenus résistent bien aux shampooings mais présentent l'inconvénient d'une mauvaise tenue si le cheveu est sensibilisé.

Il existe donc un réel besoin de mettre au point des compositions cosmétiques, pour le traitement des fibres kératiniques et en particulier la coloration des fibres kératiniques humaines telles que les cheveux et de mettre en oeuvre un procédé de coloration des cheveux qui permettent de conduire à des gainages colorés rémanents aux shampooings et aux agressions extérieures tout en conservant de bonnes propriétés cosmétiques, (apport de corps, de masse ou de volume aux cheveux), de manière durable et tout ceci quel que soit la sensibilisation des cheveux traités.

De manière surprenante et avantageuse, la demanderesse vient maintenant de découvrir que la mise en oeuvre d'une composition cosmétique pour le traitement des fibres kératiniques, en particulier des fibres kératiniques humaines, telles que les cheveux, comprenant un ou plusieurs composés organiques du silicium convenablement sélectionnés, un ou plusieurs polymères filmogènes hydrophobes, un ou plusieurs pigments et un ou plusieurs solvants volatils permettait de conduire à un gainage coloré rémanent aux shampooings ainsi qu'aux agressions extérieures tout en conservant de bonnes propriétés cosmétiques et ceci quel que soit la sensibilisation des cheveux traités.

En outre, la demanderesse a découvert qu'il était possible de mettre en oeuvre sur les fibres kératiniques, des compositions cosmétiques comprenant un ou plusieurs composés organiques du silicium tels que définis ci-après en pré-traitement d'une composition cosmétique comprenant un ou plusieurs polymères filmogènes hydrophobes, un ou plusieurs pigments et un ou plusieurs solvants volatils afin d'obtenir également un gainage coloré des cheveux ayant une tenue satisfaisante par rapport aux agressions extérieures, telles que les brushings, le frottement ou encore la transpiration.

Les gainages ainsi obtenus se présentent sous la forme d'un dépôt homogène, lisse et possède une excellente adhésion aux cheveux.

Par ailleurs, il a été constaté que les cheveux restaient parfaitement individualisés.

On entend par cheveux individualisés des cheveux qui après application de la composition et séchage ne sont pas collés (ou sont tous séparés les uns des autres) entre eux et ne forment donc pas des amas de cheveux, le gainage étant formé autour de pratiquement chaque cheveu.

De plus, il a été constaté que l'utilisation sur les fibres kératiniques d'une composition cosmétique comprenant de tels composés organiques du silicium en pré-traitement d'une composition cosmétique comprenant un ou plusieurs polymères filmogènes hydrophobes, un ou plusieurs pigments et un ou plusieurs solvants volatils permettait d'améliorer l'affinité entre les fibres kératiniques et le complexe polymère filmogène hydrophobe/pigment et ce quel que soit la sensibilisation des cheveux.

Il a été également observé que l'utilisation sur les fibres kératiniques d'une composition cosmétique comprenant un ou plusieurs composés organiques de silicium tels que définis ci-après en pré-traitement d'une composition comprenant un ou plusieurs polymères filmogènes hydrophobes, un ou plusieurs pigments et un ou plusieurs solvants volatils permettait de conférer de la masse et du corps aux cheveux et ceci de manière rémanente, en particulier vis-à-vis des shampooings.

Par ailleurs, lorsque la composition de pré-traitement comprend de l'eau ou lorsqu'elle est appliquée en présence d'eau, par exemple sur cheveux humides, le ou les composés organiques du silicium peuvent s'hydrolyser puis se condenser pour former un prépolymère hybride favorisant l'adhésion de l'organosilane sur le cheveu.

Il a été aussi observé que la tenue de la coloration aux shampooings était améliorée par rapport à celle obtenue avec des compositions cosmétiques à base de polymères filmogènes hydrophobes et de solvant volatil utilisées seules.

La présente invention a donc notamment pour objet une composition cosmétique prête à l'emploi pour le traitement des fibres kératiniques, en particulier des fibres kératiniques humaines, telles que les cheveux, comprenant :
- un ou plusieurs composés organiques du silicium choisis parmi les silanes comprenant un, deux ou trois atomes de silicium, lesdits composés organiques du silicium comportant en outre une ou plusieurs fonctions chimiques basiques et un ou plusieurs groupes hydroxyles ou hydrolysables par molécule ;
- un ou plusieurs polymères filmogènes hydrophobes,
- un ou plusieurs pigments et
- un ou plusieurs solvants volatils.

La présente invention concerne également un procédé de traitement cosmétique des cheveux, comprenant l'application sur lesdites fibres de la composition selon l'invention.

De même, la présente invention porte sur l'utilisation de la composition selon l'invention pour la coloration des fibres kératiniques, en particulier des fibres kératiniques humaines telles que les cheveux.

Par ailleurs, la présente invention porte sur l'utilisation d'une composition cosmétique comprenant, dans un milieu cosmétiquement acceptable, un ou plusieurs composés organiques du silicium, choisis parmi les silanes comprenant un, deux ou trois atomes de silicium, lesdits composés organiques du silicium comportant en outre une ou plusieurs fonctions chimiques basiques et un ou plusieurs groupes hydroxyles ou hydrolysables par molécule en pré-traitement d'une composition cosmétique contenant un ou plusieurs polymères filmogènes hydrophobes, un ou plusieurs pigments et un ou plusieurs solvants volatils.

Autrement dit, la composition cosmétique comprenant le ou les composés organiques du silicium tels que décrits ci-avant est utilisée en complément avant la composition cosmétique comprenant le ou les polymères filmogènes hydrophobes, un ou plusieurs pigments et le ou les solvants volatils.

Ainsi la composition cosmétique comprenant le ou les composés organiques du silicium tels que décrits ci-avant est utilisée pour le pré-traitement des fibres kératiniques avant l'application de la composition cosmétique comprenant le ou les polymères filmogènes hydrophobes, le ou les pigments et le ou les solvants volatils.

D'autres objets et caractéristiques, aspects et avantages de l'invention apparaîtront encore plus clairement à la lecture de la description et des exemples qui suivent.

Les composés organiques du silicium selon l'invention sont choisis parmi les organosilanes comprenant un, deux ou trois atomes de silicium, de préférence deux atomes de silicium. Ils doivent en outre comporter une ou plusieurs fonctions chimiques basiques. La fonction chimique basique peut correspondre à toute fonction conférant un caractère basique au composé de silicium et est de préférence une fonction amine telle qu'une fonction amine primaire, secondaire ou tertiaire. La fonction chimique basique des composés du silicium selon l'invention, peut comporter éventuellement d'autres fonctions, telles que, par exemple, une autre fonction amine, une fonction acide ou une fonction halogène.

Le ou les composés organiques du silicium utilisés selon invention, comportent en outre deux ou plusieurs groupes hydrolysables ou hydroxyles par molécule. Les groupes hydrolysables sont de préférence des groupes alcoxy, aryloxy ou halogène. Ils peuvent également, éventuellement, comporter d'autres fonctions chimiques telles que des fonctions acides.

Selon un mode de réalisation particulier, le ou les composés organiques du silicium utilisés selon l'invention sont choisis parmi les composés de formule (I) : 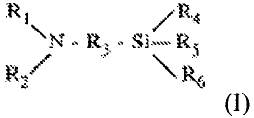 dans laquelle :
R₄ représente un halogène, un groupe OR' ou R'₁ ;
R₅ représente un halogène, un groupe OR" ou R'₂ ;
R₆ représente un halogène, un groupe OR"' ou R'₃ ;
R₁, R₂, R₃, R', R", R"', R'₁, R'₂, R'₃ représentent, indépendamment les uns des autres, un groupe hydrocarboné saturé ou insaturé, linéaire ou ramifié, portant éventuellement des groupes chimiques supplémentaires, R₁, R₂, R', R" et R"' pouvant en outre désigner l'hydrogène, et deux au moins des groupes R₄, R₅ et R₆ étant différents des groupes R'₁, R'₂ et R'₃.

De préférence, les groupes R₁, R₂, R', R'₁, R'₂, R'₃, R"et R"' sont choisis parmi les radicaux alkyle de C₁-C₁₂, aryle de C₅ à C₁₄, alkyl(C₁-C₈)aryle de C₅ à C₁₄, et aryl(C₅-C₁₄)alkyle de C₁ à C₈.

De préférence, les groupes R₁, R₂, R', R'₁, R'₂, R'₃, R"et R"' sont choisis parmi les radicaux alkylène en C₁-C₁₂, éventuellement substitué par un groupement amino, arylène en C₅-C₁₄, alkylène(C₁-C₈)arylène en C₅-C₁₄, et arylène(C₅-C₁₄)alkylène en C₁-C₈.

Selon un mode de réalisation particulier, le ou les composés organiques du silicium, présents dans la composition cosmétique prête à l'emploi ainsi que dans la composition utilisée en pré-traitement selon l'invention, répondant aux formules (I) sont le 3-aminopropyltriéthoxysilane, le 3-aminopropylméthyldiéthoxysilane, le N-(2-aminoéthyl)-3-aminopropylltriéthoxysilane et le 3-(2-aminoethylamino)propyl-méthyldiéthoxysilane.

Selon un autre mode de réalisation, le ou les composés organiques du silicium, présents dans la composition cosmétique prête à l'emploi et la composition utilisée en pré-traitement selon l'invention, sont choisis parmi les composés de formule (II) :

(R₂₁O)ₓ(R₂₂)_{y}Si - (B)ₚ - [N R₂₃ - (B')_{p'}]_{q} - [N R'₂₃ - (B")_{p"}]_{q}· - Si -(R'₂₂)_{y'}(OR'₂₁)_{x'} (II)

dans laquelle :
R₂₁, R₂₂, R'₂₁ et R'₂₂ représentent chacun indépendamment une chaîne hydrocarbonée saturée ou insaturée, linéaire ou ramifiée, contenant éventuellement un ou plusieurs hétéroatomes, éventuellement interrompue ou substituée par un ou plusieurs groupes choisis parmi les groupes éther, ester, amine, amide, carboxyle, hydroxyle et carbonyle,
x est un entier variant de 1 à 3, y = 3-x, x' est un entier variant de 1 à 3, y' = 3-x', p = 0 ou 1, p' = 0 ou 1, p" = 0 ou 1, q = 0 ou 1, q' = 0 ou 1, étant entendu que au moins q ou q' est différent de zéro,
B, B' et B" représentent chacun indépendamment un radical divalent alkylène linéaire ou ramifié en C₁-C₂₀,
R₂₃ et R'₂₃ représentent chacun indépendamment un atome d'hydrogène ou une chaîne hydrocarbonée saturée ou insaturée, linéaire ou ramifiée, contenant éventuellement un ou plusieurs hétéroatomes, éventuellement interrompue ou substituée par un ou plusieurs groupes éther, ester d'alcool en C₁-C₂₀, amine, carboxyle, alcoxysilane, aryle en C₆-C₃₀, hydroxyle ou carbonyle, ou un cycle aromatique, hétérocyclique ou non, éventuellement substitué par un ou plusieurs groupes ester d'alcool en C₃-C₂₀, amine, amide, carboxyle, alcoxysilane, hydroxyle, carbonyle ou acyle.

Comme expliqué précédemment, R₂₁, R₂₂, R'₂₁ et R'₂₂ représentent chacun indépendamment une chaîne hydrocarbonée. Par chaîne hydrocarbonée, on entend de préférence une chaîne comportant de 1 à 30 et de préférence 1 à 10 atomes de carbone.

De même, R₂₃ et R'₂₃ peuvent représenter une chaîne hydrocarbonée. Dans ce cas, on entend de préférence une chaîne comportant de 1 à 30 et de préférence 1 à 10 atomes de carbone.

De préférence, le cycle aromatique comporte de 6 à 30 atomes de carbone. Encore plus préférentiellement, il désigne un radical phényle éventuellement substitué.

De préférence, R₂₁ = R'_{21 ;} R₂₂ = R'_{22 ;} x = x' ; y = y' ; p = p' ; A = A' ; q = 1 et q' = 0.

Le ou les composés organiques du silicium de formule (II) peuvent également présenter les caractéristiques suivantes, prises seules ou en combinaison :
- R₂₁, R₂₂, R'₂₁ et R'₂₂, identiques ou différents, représentent un alkyle en C₁-C₄,
- p=p'=1 ;
- B et B', identiques ou différents représentent un alkylène linéaire en C₁-C₄ et
- R₂₃ est l'hydrogène.

Par exemple, le ou les composés organiques du silicium peuvent comprendre un substituant comprenant une fonction amine secondaire, comme la bis[3-(triéthoxysilyl)propyl]amine de formule (CH₃CH₂O)₃-Si(CH₂)₃NH(CH₂)₃SI(OCH₂CH₃)₃ proposé par la société Fluorochem, la bis [triméthoxysilylpropyl]amine de formule (CH₃O)₃-Si(CH₂)₃NH(CH₂)₃Si(OCH₃)₃ proposé par la société Gelest, la bis[méthyldiéthoxysilylpropyl]amine de formule (CH₃CH₂O)₂CH₃Si(CH₂)₃NH(CH₂)₃SiCH₃(OCH₂CH₃)₂ proposé par la société Gelest et la bis[3-triméthoxysilylpropyl]éthylènediamine de formule (CH₃O)₃Si(CH₂)₃NH(CH)₂NH(CH₂)₃Si(OCH₃)₃ proposé par la société Gelest. Parmi ces composés, on préfère la bis[3-(triéthoxysilyl)ρropyl] amine et la bis[méthyldiéthoxysilylpropyl] amine.

Selon un autre mode de réalisation de l'invention, le ou les composés organiques du silicium sont choisis parmi les composés de formule (III) : 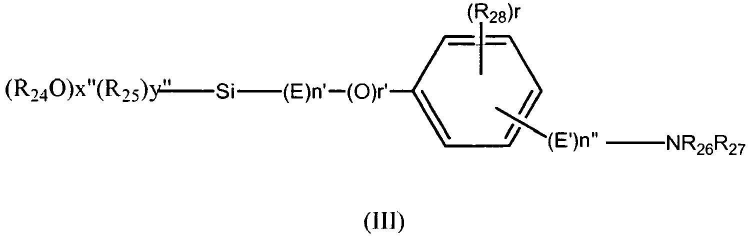 dans laquelle :
R₂₄ et R₂₅ représentent chacun indépendamment une chaîne hydrocarbonée saturée ou insaturée, linéaire ou ramifiée, contenant éventuellement un ou plusieurs hétéroatomes, éventuellement interrompue ou substituée par un ou plusieurs groupes choisis parmi les groupes éther, ester, amine, amide, carboxyle, hydroxyle et carbonyle,
x" = 2 ou 3 ;
y" = 3-x" ;
n' = 0 ou 1 ;
n" = 0 ou 1 ;
E et E' représentent chacun indépendamment un radical divalent alkylène linéaire ou ramifié en C₁-C₂₀,
R₂₆ et R₂₇ représentent chacun indépendamment l'hydrogène ou une chaîne hydrocarbonée saturée ou insaturée, linéaire ou ramifiée, contenant éventuellement un ou plusieurs hétéroatomes, éventuellement interrompue ou substituée par un ou plusieurs groupes éther, ester d'alcool en C₁-C₂₀, amine, carboxyle, alcoxysilane, aryle en C₆-C₃₀, hydroxyle ou carbonyle, ou un cycle aromatique, hétérocyclique ou non, éventuellement substitué par un ou plusieurs groupes ester d'alcool en C₁-C₂₀, amine, amide, carboxyle, alcoxysilane, hydroxyle, carbonyle ou acyle,
r est un entier variant de 0 à 4,
r' = 0 ou 1,
le ou les groupes R₂₈ représentent chacun indépendamment l'hydrogène ou une chaîne hydrocarbonée saturée ou insaturée, linéaire ou ramifiée, de préférence en C₁-C₁₀, contenant éventuellement un ou plusieurs hétéroatomes, éventuellement interrompue ou substituée par un ou plusieurs groupes éther, ester d'alcool en C₁-C₂₀, amine, carboxyle, alcoxysilane, aryle en C₆-C₃₀, hydroxyle ou carbonyle, ou un cycle aromatique, hétérocyclique ou non, éventuellement substitué par un ou plusieurs groupes ester d'alcool en C₁-C₂₀, amine, amide, carboxyle, alcoxysilane, hydroxyle, carbonyle ou acyle.

Comme expliqué précédemment, R₂₄ et R₂₅ représentent chacun indépendamment une chaîne hydrocarbonée. Par chaîne hydrocarbonée, on entend de préférence une chaîne comportant de 1 à 30 et de préférence 1 à 10 atomes de carbone.

De même, R₂₆ et R₂₇ peuvent représenter une chaîne hydrocarbonée. Dans ce cas, on entend de préférence une chaîne comportant de 1 à 30 et de préférence 1 à 10 atomes de carbone.

De préférence, le cycle aromatique comporte de 6 à 30 atomes de carbone. Encore plus préférentiellement, il désigne un radical phényle éventuellement substitué.

Le ou les composés organiques du silicium de formule (III) peuvent présenter les caractéristiques suivantes, prises seules ou en combinaison :
- R₂₄ est un alkyle en en C₁-C₄,
- x" = 3, n'= n" = 1 ; r = r' = 0,

R₂₆ et R₂₇ représentent indépendamment l'hydrogène ou un groupe choisi parmi les groupes alkyle en C₁-C₄, hydroxyalkyle en C₁-C₄ et aminoalkyle en C₁-C₄.

En particulier, le ou les composés organiques du silicium de formule (III) peuvent être choisis parmi :
- le 3-(m-aminophénoxy)propyltriméthoxysilane, de formule : 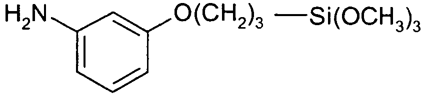
- le p-aminophényltriméthoxysilane, de formule : 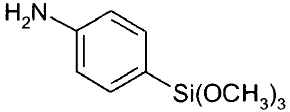
- le N-(2-aminoéthylaminométhyl)phénéthyltriméthoxysilane, de formule :

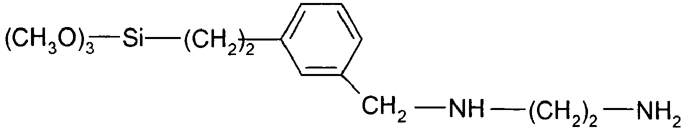

Le ou les composés organiques du silicium peuvent être présents dans la composition cosmétique prête à l'emploi et dans la composition de pré-traitement dans des proportions allant de 0,1 à 40 % en poids, plus préférentiellement de 0,2 à 20 % en poids et encore plus préférentiellement de 0,5 à 20 % en poids, par rapport au poids total de la composition.

Le ou les composés organiques de silicium peuvent être partiellement neutralisés au moyen d'un agent de neutralisation ou régulateur de pH, de telle sorte que la neutralisation atteigne 1/1000 à 99/100 et mieux de 0,2/100 à 70/100. De préférence encore, la neutralisation est de 0,2/100 à 60/100.

Les agents régulateurs de pH peuvent être tous les acides ou mélanges d'acides cosmétiquement acceptables et solubles dans le milieu de la composition. Parmi les acides utilisables, on peut citer l'acide chlorhydrique, l'acide phosphorique, l'acide sulfonique et les acides organiques. La composition utilisée selon l'invention peut également contenir un ou plusieurs autres acides organiques.

Les acides organiques sont généralement choisis parmi les acides comportant une ou plusieurs fonctions acide carboxylique, sulfonique, phosphonique ou phosphorique. Ils peuvent contenir d'autres fonctions chimiques, en particulier des fonctions hydroxy ou amino. Ils peuvent être saturés ou insaturés. On peut citer en particulier l'acide acétique, l'acide propanoïque, l'acide butanoïque, l'acide lactique, l'acide glycolique, l'acide ascorbique, l'acide maléique, l'acide phtalique, l'acide succinique, la taurine, l'acide tartrique, l'acide gluconique, l'acide glucuronique et l'acide citrique. Les acides organiques préférés sont l'acide lactique, l'acide acétique, l'acide citrique.

Cette neutralisation partielle des composés organiques du silicium peu ou pas polymérisés des compositions de l'invention revêt un aspect important pour l'obtention des propriétés voulues pour les compositions.

Par "polymère", on entend au sens de l'invention un composé correspondant à la répétition d'un ou plusieurs motifs (ces motifs étant issus de composés appelés monomères). Ce ou ces motifs sont répétés au moins deux fois et de préférence au moins 3 fois.

Par polymère hydrophobe, on entend un polymère ayant une solubilité dans l'eau à 25°C inférieure à 1% en poids.

Par polymère "filmogène", on entend un polymère apte à former à lui seul ou en présence d'un agent auxiliaire de filmification, un film macroscopiquement continu sur un support, notamment sur les matières kératiniques, et de préférence un film cohésif.

Dans un mode de réalisation préféré, le polymère organique filmogène hydrophobe est un polymère choisi parmi le groupe comprenant :
- les polymères filmogènes solubles dans un milieu solvant organique, en particulier les polymères liposolubles; cela signifie que le polymère est soluble ou miscible dans le milieu organique et va former une seule phase homogène lorsqu'il sera incorporé dans le milieu.
- les polymères filmogènes dispersibles dans un milieu solvant organique, cela signifie que le polymère forme une phase insoluble dans le milieu organique, le polymère restant stable et/ou compatible une fois incorporé dans ce milieu. En particulier de tels polymères peuvent se présenter sous la forme de dispersions non aqueuses de particules de polymères, de préférence des dispersions dans les huiles siliconées ou hydrocarbonées; dans un mode de réalisation, les dispersions non aqueuse de polymère comprennent des particules polymères stabilisées sur leur surface par au moins un agent stabilisant ; ces dispersions non aqueuses sont souvent appelées « NAD (non-aqueous dispersions) ».

- les polymères filmogènes sous forme de dispersions aqueuses de particules de polymère, cela signifie que le polymère forme une phase insoluble dans l'eau, le polymère restant stable et/ou compatible une fois incorporé dans l'eau, les particules de polymère pouvant être stabilisées sur leur surface par au moins un agent stabilisant. Ces particules de polymère sont souvent appelées « latex » ; dans ce cas, la composition doit comprendre une phase aqueuse.

Parmi les polymères filmogènes hydrophobes utilisables dans la composition de la présente invention, on peut citer les polymères synthétiques, de type radicalaire ou de type polycondensat, les polymères d'origine naturelle et leurs mélanges. Comme polymère filmogène hydrophobe, on peut citer en particulier les polymères acryliques, les polyuréthanes, les polyesters, les polyamides, les polyurées, les polymères cellulosiques comme la nitrocellulose, les polymères siliconés, les polymères et copolymères polyamide, les polyisoprènes.

Le polymère filmogène peut être choisi parmi les polymères filmogènes décrits dans la demande WO 04/028487 dont le contenu est incorporé dans la présente demande par référence.

Le polymère filmogène hydrophobe peut être notamment choisi parmi:
a) Les homopolymères et les copolymères des oléfines ; des cyclooléfines ; du butadiène ; de l'isoprène ; du styrène ; des éthers, des esters ou amides vinyliques ; des esters ou amides de l'acide (méth)acrylique contenant un groupement alkyle en C1-C20, linéaire, ramifié ou cyclique, un groupement aryle en C6-C10 ou un groupement hydroxyalkyle en C2-C6.

De tels homopolymères et copolymères peuvent être obtenus à partir de monomères choisis parmi le groupe constitué par le (méth)acrylate d'isooctyle, le (méth)acrylate d'iso-nonyle, le (méth)acrylate de 2-éthylhexyle, le (méth)-acrylate de lauryle, le (méth)acrylate d'isopentyle, le (méth)acrylate de n-butyle, le (méth)acrylate d'iso¬butyle, le (méth)acrylate d'éthyle, le (méth)acrylate de méthyle, le (méth)acrylate de tertio-butyle, le (méth)acrylate de tridécyle, le (méth)acrylate de stéaryle, le (méth)acrylate d'hydroxyéthyle, le (méth)acrylate de 2-hydroxypropyle, l'acrylate de benzyle ou de phényle. ou des mélanges de ceux-ci. Comme amides des monomères acides, on peut citer les (méth)acrylamides, et notamment les N-alkyl (méth)acrylamides, en particulier d'alkyle en C2-C12 tels que le N-éthyl acrylamide, le N-t-butyl acrylamide, le N-octyl acrylamide ; les N- dialkyl (C1-C4) (méth)acrylamides, les (méth)acrylates de perfluoroalkyle. Les polymères ci-dessus peuvent également contenir à titre de monomères de faibles quantités d'un acide carboxylique ou sulfonique insaturés tels que les acides acryliques, méthacryliques, AMPS, à condition que le caractère global du polymère demeure hydrophobe..

Comme autres monomères vinyliques utilisables, on peut encore citer :
- la N-vinylpyrrolidone, la vinylcaprolactame, les vinyl N-alkyl(C1-C6) pyrroles, les vinyl-oxazoles, les vinyl-thiazoles, les vinylpyrimidines, les vinylimidazoles,
- les oléfines tels que l'éthylène, le propylène, les butènes, l'isoprène, les butadiènes.

Le polymère vinylique peut être réticulé à l'aide d'un ou plusieurs monomères difonctionnels, notamment comprenant au moins deux insaturations éthyléniques, tel que le diméthacrylate d'éthylène glycol ou le phtalate de diallyle.

On citera, par exemple, le copolymère d'acrylate d'alkyle/acrylate de cycloalkyle commercialisé par PHOENIX CHEM. sous la dénomination GIOVAREZ AC-5099 ML, le copolymère acrylates/C12-22 alkyl methacrylate commercialisé par ROHM AND HAAS sous la dénomination SOLTEX OPT et les copolymères de vinylpyrrolidone, tels que les copolymères d'un alkène en C2 à C30, tel qu'en C3 à C22, et des associations de ceux-ci, peuvent être utilisés. Comme exemples de copolymères de VP pouvant être utilisés dans l'invention, on peut aussiciter le copolymère de VP/laurate de vinyle, de VP/stéarate de vinyle, la polyvinylpyrrolidone (PVP) butylée, de VP/hexadécène commercialisé par ISP sous la dénomination Ganex V216, de VP/eicosène commercialisé par ISP sous la dénomination Ganex V220, de VP/triacontène ou de VP/acide acrylique/méthacrylate de lauryle. On peut également citer les copolymères dont la dénomination CTFA (4ème Ed., 1991) est Octylacrylamide/acrylates/butylaminoethyl-methacrylate copolymer, tels que les produits vendus sous la dénomination AMPHOMER® ou LOVOCRYL® 47 par la société NATIONAL STARCH ainsi que les copolymères dont la dénomination CTFA est Acrylates/octylacrylamide copolymer, tels que les produits vendus sous la dénomination DERMACRYL® LT ou DERMACRYL® 79 par la société NATIONAL STARCH.

Comme polymères particuliers, on peut citer :
i) les polymères portant des groupements fluorés appartenant à l'une des classes décrites dans le texte ci-dessus, en particulier les Fomblin décrits dans le brevet US 5 948 393, les copolymères de (méth)acrylate d'alkyle/(méth)acrylate de perfluoroalkyle décrits dans les brevets EP 0 815 836 et US 5 849 318.
ii)les polymères ou copolymères résultant de la polymérisation ou la copolymérisation d'un monomère éthylénique, comprenant une ou plusieurs liaisons éthyléniques, de préférence conjuguées (ou diènes). Comme polymères ou copolymères résultant de la polymérisation ou la copolymérisation d'un monomère éthylénique, on peut utiliser des copolymères vinyliques, acryliques ou méthacryliques.

Dans un mode de réalisation, le polymère filmogène est un copolymère bloc comprenant au moins un bloc constitué de motifs styrène ou dérivés du styrène (par exemple le méthylstyrène, le chlorostyrène ou le chlorométhylstyrène). Le copolymère comprenant au moins un bloc styrène peut être un copolymère dibloc ou tribloc, voire un copolymère multibloc, en étoile ou radial. Le copolymère comprenant au moins un bloc styrène peut comprendre en outre, par exemple, un bloc alkylstyrène (AS), un bloc éthylène/butylène (EB) un bloc éthylène/ propylène (EP), un bloc butadiène (B), un bloc isoprène (I), un bloc acrylate (A), un bloc méthacrylate (MA) ou une association de ces blocs. Le copolymère comprenant au moins un bloc constitué de motifs styrène ou dérivés du styrène peut être un copolymère dibloc ou tribloc, et en particulier du type polystyrène/polyisoprène ou polystyrène/polybutadiène, tels que ceux commercialisés ou fabriqués sous la dénomination « Luvitol HSB » par BASF et ceux du type polystyrène/copoly(éthylène-propylène) ou de manière alternative du type polystyrène/copoly(éthylène/butylène), tels que ceux commercialisés ou fabriqués sous la marque de fabrique « Kraton » par Shell Chemical Co. ou Gelled Permethyl 99A par Penreco, peuvent être utilisés.

On peut citer par exemple le Kraton G1650 (SEBS), le Kraton G1651 (SEBS), le Kraton G1652 (SEBS), le Kraton G1657X (SEBS), le Kraton G1701X (SEP), le Kraton G1702X (SEP), le Kraton G1726X (SEB), le Kraton D-1101 (SBS), le Kraton D-1102 (SBS), le Kraton D-1107 (SIS), le Gelled Permethyl 99A-750, le Gelled Permethyl 99A-753-58 (mélange de polymère bloc en étoile et de polymère tribloc), le Gelled Permethyl 99A-753-59 (mélange de polymère bloc en étoile et de polymère tribloc), le Versagel MD 970 et le Versagel MD 960 de chez Penreco (mélange de polymère en étoile et de polymère tribloc dans l'isododécane).

Des copolymères de styrène-méthacrylate peuvent également être utilisés tels que les polymère commercialisés sous les référence OS 129880, OS 129881 et OS 84383 de chez Lubrizol (copolymère de styrène-méthacrylate).

Dans un mode de réalisation, le polymère filmogène est choisi parmi les copolymères d'ester vinylique (le groupe vinylique étant directement relié à l'atome d'oxygène du groupe ester et l'ester vinylique ayant un radical hydrocarboné saturé, linéaire ou ramifié, de 1 à 19 atomes de carbone, lié au carbonyle du groupe ester ) et d'au moins un autre monomère qui choisi parmi les esters vinyliques (différent de l'ester vinylique déjà présent), les α-oléfines (ayant de 8 à 28 atomes de carbone), les alkylvinyléthers (dont le groupe alkyl comporte de 2 à 18 atomes de carbone), ou les esters allyliques ou méthallyliques (ayant un radical hydrocarboné saturé, linéaire ou ramifié, de 1 à 19 atomes de carbone, lié au carbonyle du groupe ester).

Ces copolymères peuvent être partiellement réticulés à l'aide de réticulants qui peuvent être soit du type vinylique, soit du type allylique ou méthallylique, tels que le tétraallyloxyéthane, le divinylbenzène, l'octanedioate de divinyle, le dodécanedioate de divinyle, et l'octadécanedioate de divinyle.

Comme exemples de ces copolymères, on peut citer les copolymères acétate de vinyle/stéarate d'allyle, l'acétate de vinyle/laurate de vinyle, acétate de vinyle/stéarate de vinyle, acétate de vinyle/octadécène, acétate de vinyle/octadécylvinyléther, propionate de vinyle/laurate d'allyle, propionate de vinyle/laurate de vinyle, stéarate de vinyle/octadécène-1, acétate de vinyle/dodécène-1, stéarate de vinyle/éthylvinyléther, propionate de vinyle/cétyl vinyle éther, stéarate de vinyle/acétate d'allyle, diméthyl-2, 2 octanoate de vinyle/laurate de vinyle, diméthyl-2, 2 pentanoate d'allyle/laurate de vinyle, diméthyl propionate de vinyle/stéarate de vinyle, diméthyl propionate d'allyle/stéarate de vinyle, propionate de vinyle/stéarate de vinyle, réticulé avec 0,2 % de divinyl benzène, diméthyl propionate de vinyle/laurate de vinyle, réticulé avec 0,2 % de divinyl benzène, acétate de vinyle/octadécyl vinyl éther, réticulé avec 0,2 % de tétraallyloxyéthane, acétate de vinyle/stéarate d'allyle, réticulé avec 0,2 % de divinyl benzène, acétate de vinyle/octadécène-1 réticulé avec 0,2 % de divinyl benzène et propionate d'allyle/stéarate d'allyle réticulé avec 0,2 % de divinyl benzène.
iii) les polyalcènes, les copolymères d'alcènes en C2-C20, en particulier le polybutène.
iv) Les polymères d'origine naturelle, éventuellement modifiés, pouvant être choisis parmi la résine de shellac, la gomme de sandaraque, les dammars, les élémis, les copals. les polysaccharides comprenant des chaînes latérales alkyl (éther ou ester), en particulier les alkylcelluloses ayant un radical alkyle en C1 à C8 saturé ou insaturé, linéaire ou ramifié, tel que l'éthylcellulose et la propylcellulose.

Le polymère filmogène d'origine naturelle peut être choisi en particulier parmi les polymères cellulosiques tels que la nitrocellulose, l'acétate de cellulose, l'acétobutyrate de cellulose, l'acétopropionate de cellulose, On peut citer par exemple l'éthyl cellulose commercialisée par Aqualon sous la référence Aqualon Ethylcellulose N200, l'acéto-butyrate cellulose commercialisée par Eastman Chemical sous la référence CAB-381-0.5, les acéto-propionate de cellulose commercialisée par Eastman Chemical sous les références CAP-482-20 et CAP-504-0.2.

### v) les polycondensats

Parmi les polycondensats, on peut citer les polyuréthanes non ioniques, les polyuréthane-acryliques, les polyuréthanes-polyvinylpyrrolidones, les polyester-polyuréthanes, les polyéther-polyuréthanes, les polyurées, les polyurée/polyuréthanes, et des mélanges de ceux-ci.

Les polyuréthanes peuvent être par exemple un copolymère de polyuréthane aliphatique, cycloaliphatique ou aromatique, de polyurée/polyuréthane.

Les polyuréthanes tels que définis dans l'invention peuvent également être obtenus à partir de polyesters ramifiés ou non ramifiés ou à partir d'alkydes comprenant des hydrogènes mobiles qui sont modifiés au moyen d'une polyaddition avec un diisocyanate et un composé co-réactif bifonctionnel organique (par exemple dihydro, diamino ou hydroxy-amino).

On peut également citer les polyesters, les polyesteramides, les polyesters à chaîne grasse, les polyamides et les résines d'époxyester.

Les polyesters peuvent être obtenus de manière connue au moyen de la polycondensation de diacides aliphatiques ou aromatiques avec des diols aliphatiques ou aromatiques ou avec des polyols. L'acide succinique, l'acide glutarique, l'acide adipique, l'acide pimélique, l'acide subérique ou l'acide sébacique peuvent être utilisés comme diacides aliphatiques. L'acide téréphtalique ou l'acide isophtalique, voire un dérivé tel que l'anhydride phtalique, peuvent être utilisés comme diacides aromatiques. L'éthylène glycol, le propylène glycol, le diéthylène glycol, le néopentyl glycol, le cyclohexanediméthanol et le 4,4-N-(1-méthylpropylidène)bisphénol, peuvent être utilisés comme diols aliphatiques.

Les polyesteramides peuvent être obtenus de manière analogue aux polyesters, au moyen de la polycondensation de diacides avec des aminoalcools. Les polyamides peuvent être obtenus de manière analogue aux polyesters, au moyen de la polycondensation de diacides avec des diamines.

A titre de polyester particulier, on peut citer les polyesters aliphatiques ayant des chaînes latérales alkyle en C4-50 ou bien les polyesters résultant de la condensation de dimères d'acides gras, voire les polyesters comprenant un segment siliconé sous la forme d'une séquence, greffon ou groupement terminal, tel que défini dans la demande de brevet FR 0 113 920.

### b) Composés siliconés.

Le polymère filmogène hydrophobe peut aussi être un polymère comprenant au moins une partie siliconée.

Dans ce qui suit, on entend désigner par silicone ou polysiloxane, en conformité avec l'acception générale, tous les polymères ou oligomères organosiliciés à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane ≡Si-O-Si≡), des radicaux hydrocarbonés éventuellement substitués étant directement liés par l'intermédiaire d'un atome de carbone sur lesdits atomes de silicium. Les radicaux hydrocarbonés les plus courants sont les radicaux alkyls notamment en C₁-C₁₀ et en particulier méthyle, les radicaux fluoroalkyls, les radicaux aryls et en particulier phényle, et les radicaux alcényls et en particulier vinyle ; d'autres types de radicaux susceptibles d'être liés soit directement, soit par l'intermédiaire d'un radical hydrocarboné, à la chaîne siloxanique sont notamment l'hydrogène, les halogènes et en particulier le chlore, le brome ou le fluor, les thiols, les radicaux alcoxy, les radicaux polyoxyalkylènes (ou polyéthers) et en particulier polyoxyéthylène et/ou polyoxypropylène, les radicaux hydroxyls ou hydroxyalkyls, les groupements aminés substitués ou non, les groupements amides, les radicaux acyloxy ou acyloxyalkyls, les radicaux hydroxyalkylamino ou aminoalkyls, des groupements ammonium quaternaires, des groupements amphotères ou bétaïniques, des groupements anioniques tels que carboxylates, thioglycolates, sulfosuccinates, thiosulfates, phosphates et sulfates, cette liste n'étant bien entendu nullement limitative (silicones dites "organomodifiées").

Comme polymère filmogène hydrophobe comprenant au moins une partie siliconée on peut notamment citer :
i) les résines de silicone, généralement solubles ou gonflables dans les huiles de silicone.

Ces résines sont des polymères de polyorganosiloxanes réticulés.
- La nomenclature des résines de silicone est connue sous le nom de "MDTQ", la résine étant décrite en fonction des différentes unités monomèriques siloxane qu'elle comprend, chacune des lettres "MDTQ" caractérisant un type d'unité.
- La lettre M représente l'unité monofonctionelle de formule (CH₃)₃SiO_{1/2}, l'atome de silicium étant relié à un seul atome d'oxygène dans le polymère comprenant cette unité.

La lettre D signifie une unité difonctionnelle (CH₃)₂SiO_{2/2} dans laquelle l'atome de silicium est relié à deux atomes d'oxygène

La lettre T représente une unité trifonctionnelle de formule (CH₃)SiO_{3/2} .

Dans les motifs M, D, T définis précédemment, au moins un des groupes méthyles peut être substitués par un groupe R différent du groupe méthyle tel qu'un radical hydrocarboné (notamment alkyle) ayant de 2 à 10 atomes de carbone ou un groupe phényl ou bien encore un groupe hydroxyle.

Enfin, la lettre Q signifie une unité tetrafonctionnelle SiO_{4/2} dans laquelle l'atome de silicium est lié à quatre atomes d'hydrogène eux mêmes liés au reste du polymère.

Divers résines de propriétés différentes peuvent être obtenues à partir de ces différentes unités, les propriétés des ces polymères variant en fonction du type de monomères (ou unités), du type et du nombre de radicaux subsitués, de la longueur de la chaîne polymérique, du degré de ramification et de la taille des chaines pendantes.

A titre d'exemple de ces résines silicones, on peut citer :
- les siloxysilicates qui peuvent être des triméthylsiloxysilicate de formule [(CH₃)₃XSiXO]ₓX(SiO_{4/2})_{y} (unités MQ) dans laquelle x et y sont des entiers allant de 50 à 80,
- les polysilesquioxanes de formule (CH₃SiO_{3/2})_{.x} (unités T) dans laquelle x est supérieur à 100 et dont au moins un des radicaux méthyle peut être substitué par un groupement R tel que défini plus haut,
- les polyméthylsilsesquioxanes qui sont des polysilsesquioxanes dans lesquels aucun des radicaux méthyle n'est substitué par un autre groupement. De tels polyméthylsilsesquioxanes sont décrits dans le document US 5,246,694 dont le contenu est incorporé par référence.

A titre d'exemples de résines polyméthylsilsesquioxanes commercialement disponibles, on peut citer celles qui sont commercialisés :
- par la société Wacker sous la référence Resin MK tels que la Belsil PMS MK : polymère comprenant des unités répétitives CH₃SiO_{3/2} (unités T), pouvant aussi comprendre jusqu'à 1% en poids d'unités (CH₃)₂SiO_{2/2} (unités D) et présentant un poids moléculaire moyen d'environ 10000,
- par la société SHIN-ETSU sous les références KR-220L qui sont composés d'unités T de formule CH₃SiO_{3/2} et ont des groupes terminaux Si-OH (silanol), sous la référence KR-242A qui comprennent 98% d'unités T et 2% d'unités diméthyle D et ont des groupes terminaux Si-OH ou encore sous la référence KR-251 comprenant 88% d'unités T et 12% d'unités dimethyl D et ont des groupes terminaux Si-OH.

Comme résines siloxysilicates, on peut citer les résines triméthylsiloxysilicate (TMS) éventuellement sous forme de poudres. De telles résines sont commercialisées sous la référence SR1000 par la société General Electric ou sous la référence TMS 803 par la société Wacker. On peut encore citer les résines timéthylsiloxysilicate commercialisées dans un solvant tel que la cyclométhicone, vendues sous la dénomination "KF-7312J" par la société Shin-Etsu, "DC 749", "DC 593" par la société Dow Corning.
ii) Les polyamides siliconés du type polyorganosiloxane tels que ceux décrits dans les documents US A 5 874 069, US A 5 919 441, US A 6 051 216 et US A 5 981 680, dont le contenu est incorporé dans la présente demande par référence.
iii) Les composés siliconés greffés

Les compositions de l'invention peuvent aussi contenir un polymère siliconé greffé. Dans le cadre de l'invention, on entend par polymère siliconé greffé, un polymère comprenant une portion polysiloxane et une portion constituée d'une chaîne organique non-siliconée, l'une des deux portions constituant la chaîne principale du polymère l'autre étant greffée sur ladite chaîne principale.

Les polymères siliconés greffés utilisés dans les compositions cosmétiques selon l'invention sont choisis préférentiellement dans le groupe constitué par les polymères à squelette organique non siliconé greffé par des monomères contenant un polysiloxane, les polymères à squelette polysiloxanique greffé par des monomères organiques non siliconés et leurs mélanges.

Les monomères organiques non siliconés constituant la chaîne principale du polymère siliconé greffé peuvent être choisis parmi des monomères à insaturation éthylénique polymérisables par voie radicalaire, des monomères polymérisables par polycondensation tels que ceux formant des polyamides, des polyesters, des polyuréthanes, des monomères à ouverture de cycle tels que ceux du type oxazoline ou caprolactone.

Les polymères à squelette organique non-siliconé greffé par des monomères contenant un polysiloxane, conformes à l'invention, peuvent être choisis parmi ceux décrits dans les brevets US 4,693,935, US 4,728,571 et US 4,972,037 et les demandes de brevet EP-A-0 412 704, EP-A-0 412 707,EP-A-0 640 105 et WO 95/00578. Il s'agit de copolymères obtenus par polymérisation radicalaire à partir de monomères à insaturation éthylénique et de macromères siliconés ayant un groupe vinylique terminal ou bien des copolymères obtenus par réaction d'une polyoléfine comprenant des groupes fonctionnalisés et d'un macromère polysiloxane ayant une fonction terminale réactive avec lesdits groupes fonctionnalisés.

Le copolymère à squelette organique non-siliconé greffé par des monomères contenant un polysiloxane peut par exemple avoir la structure suivante :

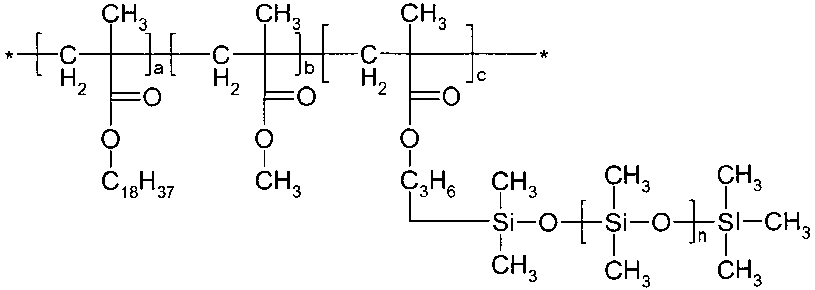

Un tel polymère est commercialisé sous le nom KP 561 commercialisés par Shin Etsu.

Le copolymère à squelette organique non-siliconé greffé par des monomères contenant un polysiloxane peut aussi avoir la structure suivante :

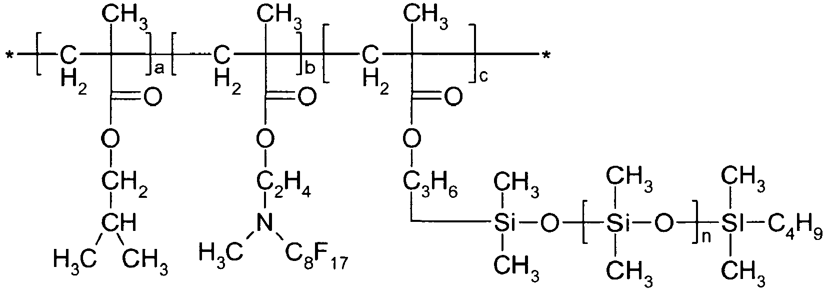

Un tel polymère, le Polysilicone 7, est commercialisé sous le nom SA70 par 3M.

D'autres copolymères à squelette organique non-siliconé greffé par des monomères contenant un polysiloxane peuvent aussi être le KP545, le KP574 et le KP575 commercialisés par Shin Etsu.

Comme composé siliconé greffé, on peut également citer le copolymère d'isobutylmethacrylate / bis-hydroxypropyl dimethicone acrylate commercialisé par Grant Industrie sous le nom Granacrysil BMAS.

Selon la présente invention, le ou les polymères siliconés greffés, à squelette polysiloxanique greffé par des monomères organiques non siliconés comprennent une chaîne principale de silicone (ou polysiloxane (≡Si-O-)ₙ) sur laquelle se trouve greffé, à l'intérieur de ladite chaîne ainsi qu'éventuellement à l'une au moins de ses extrémités, au moins un groupement organique ne comportant pas de silicone.

Des exemples de polymères siliconés correspondant à la définition sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle. Comme composé répondant à cette définition on peut citer le poly dimethyl / methyl siloxane à groupements propyl thio-3 acrylate de methyle / methacrylate de methyle / acide methacrylique ou Polysilicone-8 commercialisé sous le nom VS80 par la société 3M.

D'autres exemples de polymères siliconés sont notamment des polydiméthylsiloxanes (PDMS) sur lesquels sont greffés, par l'intermédiaire d'un chaînon de raccordement de type thiopropylène, des motifs polymères du type poly(méth)acrylate d'isobutyle.

De préférence, la masse moléculaire en nombre des polymères siliconés à squelette polysiloxanique greffé par des monomères organiques non siliconés de l'invention varie de 10 000 à 1 000 000 environ, et encore plus préférentiellement de 10 000 à 100 000 environ.

De préférence, les polymères siliconés greffés sont choisis dans le groupe constitué par le copolymère de méthacrylate d'alkyles greffé polydiméthylsiloxane, les copolymères de méthacrylate d'isobutyle, d'acide acrylique et de macromère siliconé et le poly diméthyl / méthyl siloxane à groupements propyl thio-3-acrylate de méthyle / méthacrylate de méthyle / acide méthacrylique.

### iv) Les silicone polyurée/uréthane

Le copolymère de l'invention peut comprendre en plus du polysiloxane/polyurée d'autres blocs de motifs différents. On citera en particulier les terpolymères blocs polysiloxane/polyurée/polyuréthane.

Selon une variante, le copolymère contient uniquement un ou plusieurs blocs siloxane et un ou plusieurs blocs polyurée.

Selon l'invention, le copolymère peut répondre à la formule générale (IV) : 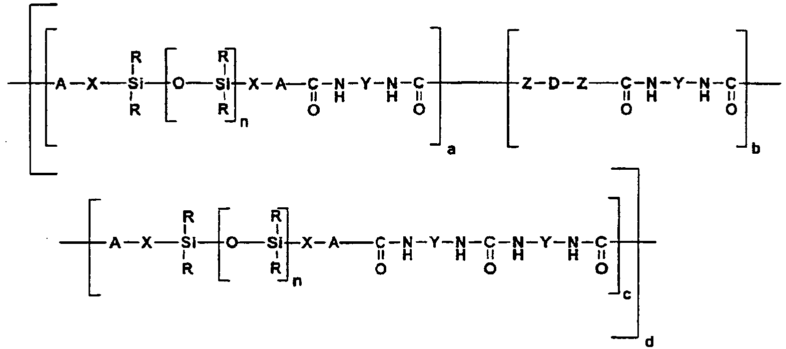 dans laquelle
R représente un radical hydrocarboné monovalent, le cas échéant substitué par le fluor ou le chlore, ayant 1 à 20 atomes de carbone,
X représente un radical alkylène ayant 1 à 20 atomes de carbone, dans lequel des unités méthylène non voisines peuvent être remplacées par des radicaux -O-,
A représente un atome d'oxygène ou un radical amino -NR'-,
Z représente un atome d'oxygène ou un radical amino -NR'-,
R' représente hydrogène ou un radical alkyle ayant 1 à 10 atomes de carbone,
Y représente un radical hydrocarboné bivalent, le cas échéant substitué par le fluor ou le chlore, ayant 1 à 20 atomes de carbone,
D représente un radical alkylène, le cas échéant substitué par fluor, chlore, alkyle en C₁-C₆ ou ester d'alkyle en C₁-C₆, ayant 1 à 700 atomes de carbone, dans lequel des unités méthylène non voisines peuvent être remplacées par des radicaux -O-, -COO-, -OCO- ou - OCOO-,
   - n: est un nombre allant de 1 à 4000,
   - a: est un nombre d'au moins 1,
   - b: est un nombre allant de 0 à 40,
   - c: est un nombre allant de 0 à 30, et
   - d: est un nombre supérieur à 0.
à la condition que A représente dans au moins un des motifs (a) un radical NH

De préférence, R représente un radical hydrocarboné monovalent avec 1 à 6 atomes de carbone par exemple méthyle, éthyle, vinyle et phényle. Selon un mode de réalisation particulier, R est un radical alkyle non substitué.

De préférence, X représente un radical alkylène avec 2 à 10 atomes de carbone. De préférence, le radical alkylène X n'est pas interrompu.

Selon un mode de réalisation particulier, le groupement A dans tous les motifs (b) et (c), lorsqu'ils sont présents, représente NH.

Selon un mode de réalisation particulièrement préféré, tous les groupements A représentent un radical NH.

De préférence, Z représente un atome d'oxygène ou un radical NH.

De préférence, Y représente un radical hydrocarboné comprenant de 3 à 13 atomes de carbone, qui est de préférence, non substitué. De préférence, Y représente un radical aralkylène, alkylène, linéaire ou cyclique.

De préférence, D représente un radical alkylène avec au moins 2, en particulier au moins 4 atomes de carbone et au maximum 12 atomes de carbone.

De préférence également, D représente un radical polyoxyalkylène, en particulier un radical polyoxyéthylène ou polyoxypropylène avec au moins 20, en particulier au moins 100 atomes de carbone et au maximum 800, en particulier au maximum 200 atomes de carbone.

De préférence, le radical D n'est pas substitué.

De préférence, n représente un nombre d'au moins 3, en particulier au moins 25 et de préférence, au maximum 800, en particulier au maximum 400, de manière particulièrement préférée, au maximum 250.

De préférence, a représente un nombre de plus de 50.

Lorsque b est différent de 0, b représente de préférence, un nombre d'au maximum 50, en particulier au maximum 25.

De préférence, c représente un nombre d'au maximum 10, en particulier au maximum 5.

Les copolymères de l'invention peuvent être obtenus selon les procédés de polymérisation décrits dans la demande de brevet US 2004/0254325 ou la demande WO 03/014194.

Selon un autre mode de réalisation, le copolymère est un copolymère polysiloxane/polyurée non ionique, c'est-à-dire qu'il ne contient pas de groupement ionisé ou ionisable.

A titre d'exemple de copolymère, on peut citer le copolymère diméthylpolysiloxane/urée, de nom INCI polyureadimethicone.

Un tel polymère peut être obtenu notamment par copolymérisation d'un alpha,oméga-aminosilicone avec un diisocyanate. Des polymères répondant à ces caractéristiques sont par exemple les produits commercialisés sous la référence Wacker-Belsil ® UD 60 Wacker-Belsil ® UD 80, Wacker-Belsil ® UD 140 et Wacker-Belsil ® UD 200 par la société Wacker.

### v) Les copolymères à base de résine de silicone et de silicone fluide.

Ces copolymères siliconés sont obtenus par réaction d'une résine de silicone et d'une silicone fluide.

De tels copolymères sont décrits par exemple dans « Silicone Pressure Sensitive Adhesive », Sobieski and Tangney, Handbook of Pressure Sensitive Adhesive Technology (D. Satas Ed.), Von Nostrand Reinhold, New York.

Dans le copolymère, la résine de silicone est présente à un taux compris entre 45 et 75% (par rapport à la masse totale de silicone) et la silicone fluide est présente à un taux compris entre 25 et 55%, avec la somme des pourcentages de résine de silicone et de silicone fluide qui est égal à 100. De préférence, la résine de silicone est présente à un taux compris entre 55 et 65% (par rapport à la masse totale de silicone) et la silicone fluide est présente à un taux compris entre 35 et 45%, avec la somme des pourcentages de résine de silicone et de silicone fluide qui est égal à 100.

De préférence, la résine de silicone selon l'invention est le produit de condensation de groupements SiO₂ et de groupements R₃(SiO)_{1/2} (triorganosilyl) pour lequel chaque groupement R est indépendamment sélectionné parmi les radicaux méthyle, éthyle, propyle ou vinyl et pour lequel le rapport entre les fonctions SiO2 et les fonctions R₃(SiO)_{1/2} de la résine de silicone va de 0.6 à 0.9. Des groupements triorganosilyl utilisables pour former la résine de silicone peuvent être des unités triméthylsilyl, triéthylsilyl, méthylméthylproprylsilyl, diméthylvinylsilyl et des mélanges de celles-ci. Le groupement triméthylsilyl est le préféré dans le cadre de l'invention.

De préférence, la silicone fluide selon l'invention est une diorganopolysiloxane aux fonctions OH terminales ayant une viscosité comprise entre 100 et 100.000 est à 25°C pour laquelle les substituants du diorganopolysiloxane sont indépendamment choisis parmi les radicaux méthyle, éthyle, propyle ou vinyl. Les diorganosiloxances sont de préférence des polymères linéaires. Des exemples de diorganopolysiloxane peuvent être, de manière non limitative, une polydiméthylsiloxane, une éthylméthylpolysiloxane, un copolymère de diméthylsiloxane et de méthylvinylsiloxane, et des mélanges de tels polymères ou copolymères ayant des extrémités OH. Le diorganopolysiloxane préféré est une polydiméthylsiloxane.

Des exemples de synthèse d'un tel copolymère sont par exemple décrits dans le brevet US5162410 ou dans le brevet CA711756.

Les copolymères préférés selon l'invention sont commercialisés par Dow Corning sous la référence BIO-PSA®, ces BIO-PSA® pouvant être sous deux formes, standard ou amine compatible, et étant fournis dans différents solvants avec plusieurs ratio résine de silicone/silicone fluide. On peut notamment citer les grades 7-4400, 7-4500, 7-4600. Le BIO-PSA® particulièrement préféré selon l'invention est le grade 7-4400.

Lorsque le polymère filmogène selon l'invention est dispersé dans le solvant organique, les compositions selon l'invention comprennent avantageusement au moins une dispersion stable de particules de polymère essentiellement sphériques d'un ou plusieurs polymères. Avant leur incorporation dans la composition de l'invention, les particules sont généralement dispersées dans une phase grasse liquide physiologiquement acceptable, telle que les huiles hydrocarbonées ou les huiles siliconées. Selon un mode de mise en oeuvre, ces dispersions sont généralement connues comme « NAD » (dispersions non aqueuses) de polymère par opposition aux réseaux qui sont des dispersions aqueuses de polymère.

Ces dispersions peuvent notamment se présenter sous forme de nanoparticules de polymères en dispersion stable dans ladite phase organique liquide. Les nanoparticules sont de préférence d'une taille moyenne comprise entre 5 et 800 nm, et mieux entre 50 et 500 nm. Il est toutefois possible d'obtenir des tailles de particules de polymère allant jusqu'à 1 µm.

Les polymères en dispersion pouvant être utilisés dans les compositions de l'invention ont préférablement un poids moléculaire allant d'environ 2000 à 10.000.000 et une Tg allant de -100°C à 300°C et préférablement de -10°C à 80°C.

Parmi les polymères filmogènes en dispersion, on peut citer des homopolymères ou des copolymères radicalaires, acryliques ou vinyliques, de préférence ayant une Tg inférieure ou égale à 40°C et notamment allant de - 10° à 30°C, utilisés seul ou en mélange.

Selon un mode de mise en oeuvre, les particules de polymère sont stabilisées par un stabilisant solide à température ambiante, qui peut être un polymère séquencé, un polymère greffé, et/ou un polymère statistique, seul ou en mélange. La stabilisation peut être effectuée par tout moyen connu, et en particulier par ajout direct du polymère stabilisant lors de la polymérisation.

Lorsqu'une dispersion aqueuse de particules polymères est utilisée, la teneur en matière sèche de ladite dispersion aqueuse peut être de l'ordre de 3 à 60% en poids, et préférablement de 10 à 50%.

La taille des particules polymères en dispersion aqueuse peut être comprise entre 10 et 500 nm, et elle est préférablement comprise entre 20 et 150 nm, permettant l'obtention d'un film ayant un brillant notable. Cependant, on peut utiliser des tailles de particules allant jusqu'à un micron.

De façon non limitative, les polymères filmogènes hydrophobes préférés sont choisis parmi les polyuréthanes ; polyuréthanes-acryliques ; les polyurées ; les polyurée-polyuréthanes ; les polyester-polyuréthanes ; les polyéther-polyuréthanes ; les polyesters ; les polyesters amides ; les polymères ou copolymères acryliques et/ou vinyliques ; les polyacrylamides ; les polyester acryliques ; les polymères ou copolymères à base de polyvinylpyrrolidone ; les polymères siliconés ; les polymères siliconés comprenant des parties polyuréthanes, polyurées ou acryliques ; les résines de silicone ; les copolymères à base de résine de silicone et de diméthiconol ; les polymères fluorés ; les celluloses et leurs mélanges. Selon un mode de réalisation particulier, le polymère filmogène hydrophobe est choisi parmi les polymères ou copolymères acryliques, les polyester acryliques, les polymères ou copolymères à base de polyvinylpyrrolidone, les résines de silicone, les copolymères à base de résine de silicone et de diméthiconol, les polymères siliconés comprenant des parties polyuréthanes, polyurées ou acryliques ; les celluloses et leurs mélanges.

Les polymères filmogènes hydrophobes selon l'invention peuvent être sélectionnés sur leurs propriétés mécaniques. De telles propriétés peuvent être la flexibilité, la dureté, l'adhésion, lé rémanence, la résistance à l'eau ou à d'autres composés chimiques, la résistance à l'abrasion. Il est aussi possible de prendre avantage des propriétés plus versatiles des polymères blocs (polymères constitués de deux ou plus segments de polymères distincts), des polymères greffés (polymères ayant une chaîne pendante polymérique greffée sur le squelette de l'homopolymère ou du copolymère), des hétéropolymères (polymères comprenant deux ou plus monomères différents). Dans les copolymères par exemple, la quantité de blocs durs et mous ont un impact significatif sur les propriétés du polymère.

De plus il est possible de mélanger deux polymères ou plus pour parvenir à la propriété souhaitée. Des exemples de combinaisons peuvent être polyuréthanes et polyacrylates, polyuréthane et polyesters, deux polymères ayant une partie siliconée, polyuréthane et polymère ayant une partie siliconée.

Selon un mode de réalisation particulier, le polymère filmogène hydrophobe est un polymère non ionique. Selon un autre mode de réalisation, le polymère filmogène est solide à 25°C, en ce sens que l'on n'observe pas son écoulement à l'oeil nu au bout d'une durée d'une heure.

Le polymère filmogène hydrophobe peut être présent dans les compositions selon l'invention en une teneur allant de 0,1 % à 40 % en poids, par rapport au poids total de la composition, de préférence allant de 0,1 % à 30 % en poids, de préférence allant de 0,5 à 20 % en poids, préférentiellement allant de 1 % à 20 % en poids, et plus préférentiellement allant de 1 % à 15 % en poids.

Lorsque le polymère a une température de transition vitreuse trop élevée pour l'utilisation désirée, on peut y associer un plastifiant de façon à abaisser cette température du mélange utilisé. Le plastifiant peut être choisi parmi les plastifiants utilisés habituellement dans le domaine d'application, et notamment parmi les composés pouvant être des solvants pour le polymère.

De préférence, le plastifiant a une masse moléculaire inférieure ou égale à 5000 g/mol, de préférence inférieure ou égale à 2000 g/mol, préférentiellement inférieure ou égale à 1000 g/mol, et plus préférentiellement inférieure ou égale à 900 g/mol. Le plastifiant a avantageusement une masse moléculaire supérieure ou égale à 100 g/mol.

Ainsi, les compositions cosmétiques selon l'invention peuvent comprendre, en outre, un ou plusieurs agents plastifiants. En particulier, on peut citer, seuls ou en mélange, les plastifiants usuels, tels que :
- les glycols et leurs dérivés, tels que le diéthylène glycol éthyléther, le diéthylène glycol méthyléther, le diéthylène glycol butyléther ou encore le diéthylène glycol hexyléther, l'éthylène glycol éthyléther, l'éthylène glycol butyléther, l'éthylène glycol hexyléther ;
- les polyéthylène glycols, les polypropylène glycols, les copolymères polyéthylène glycols-polypropylène glycols et leurs mélanges, notamment les polypropylène glycols de haut poids moléculaire, ayant par exemple une masse moléculaire allant de 500 à 15000, tels que par exemple
- les esters de glycol ;
- les dérivés de propylène glycol et en particulier le propylène glycol phényléther, le propylène glycol diacétate, le dipropylène glycol éthyléther, le tripropylène glycol méthyléther et le diéthylène glycol méthyléther, le dipropylène glycol butyléther. De tels composés sont commercialisés par Dow Chemical sous les dénominations Dowanol PPH et Dowanol DPnB.
- des esters d'acides, notamment carboxyliques, tels que des citrates, des phtalates, des adipates, des carbonates, de tartrates, des phosphates, des sébaçates ;
- les esters issus de la réaction d'un acide monocarboxylique de formule R₁₁COOH avec un diol de formule HOR₁₂OH avec R₁₁ et R₁₂, identiques ou différents, représentent une chaîne hydrocarbonée, comprenant de préférence de 3 à 15 atomes de carbone, linéaire, ramifiée ou cyclique, saturée ou insaturée comprenant éventuellement un ou plusieurs hétéroatomes tels que N, O , S, en particulier le monoester résultant de la réaction de l'acide isobutyrique et d'octanediol tel que le triméthyl-2,2,4 pentane diol 1,3, tel que celui commercialisé sous la référence TEXANOL Ester Alcohol par la société Eastman Chemical,
- des dérivés oxyéthylénés, tels que les huiles oxyéthylénées, notamment les huiles végétales, telles que l'huile de ricin ;
- leurs mélanges.

Plus particulièrement, le plastifiant peut être choisi parmi les esters d'au moins un acide carboxylique comprenant 1 à 7 atomes de carbones et d'un polyol comprenant au moins 4 groupes hydroxyles.

Le polyol selon l'invention peut être un ose - polyhydroxyaldéhyde (aldose) ou polyhydroxycétone (cétose) - cyclisé ou non. Le polyol est de préférence un ose cyclisé sous forme d'hémi-acétal.

Le polyol peut être un mono- ou un polysaccharide comprenant de 1 à 10 oses, de préférence de 1 à 4, de préférence encore un ou deux oses. Le polyol peut être choisi parmi l'érythritol, le xylitol, le sorbitol, le glucose, le saccharose, le lactose, le maltose.

Le polyol selon l'invention est de préférence un disaccharide. Parmi les disaccharides, on peut citer le saccharose (appelé également alpha-D-glucopyranosyl-( 1-2)-béta-D-fructofuranose), le lactose (appelé également béta-D-galactopyranosyl-(1-4)-béta-D-glucopyranose) et le maltose (appelé également alpha-D-glucopyranosyl-(1-4)-béta-D-glucopyranose), et de préférence le saccharose.

L'ester selon l'invention peut être constitué d'un polyol estérifié par au moins deux acides monocarboxyliques différents, ou par au moins trois acides monocarboxyliques différents.

L'ester selon l'invention peut être un copolymère de deux esters, en particulier un copolymère i) d'un saccharose substitué par des groupements benzoyle et ii) d'un saccharose substitué par des groupements acétyle et/ou isobutyryle.

L'acide carboxylique est de préférence un acide monocarboxylique comprenant de 1 à 7 atomes de carbones, de préférence de 1 à 5 atomes de carbone, par exemple choisi parmi les acides acétique, n-propanoïque, isopropanoïque, n-butanoïque, isobutanoïque, tertiobutanoïque, n-pentanoïque et benzoïque.

L'ester peut être obtenu à partir d'au moins deux acides monocarboxyliques différents. Selon un mode de mise en oeuvre, l'acide est un acide linéaire ou ramifié non substitué.

L'acide est de préférence choisi parmi l'acide acétique, l'acide isobutyrique, l'acide benzoïque, et leurs mélanges, et plus préférentiellement .

Selon un mode de mise en oeuvre préféré, l'ester est le diacétate hexa-(2-méthyl-propanoate) de saccharose, tel que celui vendu sous la dénomination "Sustane SAIB Food Grade Kosher" par la société EASTMAN CHEMICAL.

Selon un autre mode de réalisation, le plastifiant peut être choisi parmi les esters d'acide polycarboxylique aliphatique ou aromatique et d'alcool aliphatique ou aromatique comprenant de 1 à 10 atomes de carbone.

L'alcool aliphatique ou aromatique comprend de 1 à 10 atomes de carbone, de préférence de 1 à 8, par exemple de 1 à 6. Il peut être choisis parmi les alcools R1OH, tels que R1 représente méthyle, éthyle, propyle, isopropyle, butyle, hexyle, éthylhexyle, décyle, isodécyle, benzyle, ou benzyle substitué par un alkyle comprenant 1 à 3 atomes de carbone, et leurs mélanges.

L'acide polycarboxylique aliphatique ou aromatique comprend de préférence de 3 à 12 atomes de carbone, de préférence de 3 à 10 atomes de carbone, de préférence de 3 à 8 atomes de carbone, par exemple 6 ou 8 atomes de carbones.

L'acide polycarboxylique aliphatique ou aromatique est avantageusement choisi parmi les acides dicarboxyliques et les acides tricarboxyliques.

Parmi les acides dicarboxyliques aliphatiques, on peut citer ceux de formule HOOC-(CH2)n-COOH, dans laquelle n est un entier allant de 1 à 10, de préférence allant de 2 à 8, par exemple égal à 2, 4, 6 ou 8.

On préfère les acides dicarboxyliques choisis parmi l'acide succinique, l'acide adipique et l'acide sébacique.

Parmi les acides dicarboxyliques aromatiques, on peut citer l'acide phtalique.

Parmi les acides tricarboxyliques, on peut citer les triacides qui correspondent à la formule 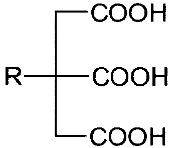 dans laquelle R représente un groupement -H, -OH ou -OCOR' dans lequel R' représente un groupement alkyle ayant de 1 à 6 atomes de carbone. De préférence, R représente un groupement -OCOCH3.

L'acide tricarboxylique est notamment choisi parmi l'acide acétyl-citrique, l'acide butyroyl-citrique, l'acide citrique.

Parmi les esters d'acide tricarboxylique, on peut utiliser les esters dérivés de l'acide citrique ( ou citrates) tels que l'acétyl-citrate de tributyle, l'acétyl-citrate de triéthyle, l'acétyl-citrate de triéthylhexyle, l'acétyl-citrate de trihexyle, le butyroyl- citrate de trihexyle, le citrate de triisodécyle, le citrate de triisopropyle, le citrate de tributyle et le citrate de tri(éthyl-2- hexyle). Comme références commerciales de plastifiants mentionnés ci-dessus on peut citer, la gamme Citroflex commercialisée par Vertellus avec notamment le Citroflex A4 et le Citroflex C2.

Parmi les esters de l'acide adipique, on peut citer l'adipate de dibutyle et l'adipate de di(éthyl-2-hexyle).

Parmi les esters de l'acide sébacique, on peut citer le sébaçate de dibutyle, le sébaçate de di(éthyl-2-hexyle), le sébaçate de diéthyle et le sébaçate de diisopropyle.

Parmi les esters de l'acide succinique, on peut citer le succinate de di(éthyl-2-hexyle) et le succinate de diéthyle.

Parmi les esters de l'acide phtalique, on peut citer le phtalate de butyle et de benzyle, le phtalate de dibutyle, le phtalate de diéthylhexyle, le phtalate de diéthyle et le phtalate de diméthyle.

Avantageusement, le plastifiant peut être présent dans les compositions en une teneur telle que le rapport massique entre le polymère filmogène hydrophobe et le plastifiant est compris entre 0,5 et 100, de préférence entre 1 et 50, de préférence entre 1 et 10.

La composition cosmétique prête à l'emploi et la composition cosmétique comprenant le ou les polymères filmogènes hydrophobes selon l'invention comprennent un ou plusieurs pigments.

Par pigment, on entend tous les pigments apportant de la couleur aux matières kératiniques. Leur solubilité dans l'eau à 25 °C et à pression atmosphérique (760 mmHg) est inférieure à 0,05 %, et de préférence inférieure à 0,01 %.

Les pigments qui peuvent être utilisés sont notamment choisis parmi les pigments organiques et/ ou minéraux connus de la technique, notamment ceux qui sont décrits dans l'encyclopédie de technologie chimique de Kirk-Othmer et dans l'encyclopédie de chimie industrielle de Ullmann.

Ces pigments peuvent se présenter sous forme de poudre ou de pâte pigmentaire. Ils peuvent être enrobés ou non enrobés.

Les pigments peuvent par exemple être choisis parmi les pigments minéraux, les pigments organiques, les laques, les pigments à effets spéciaux tels que les nacres ou les paillettes, et leurs mélanges.

Le pigment peut être un pigment minéral. Par pigment minéral, on entend tout pigment qui répond à la définition de l'encyclopédie Ullmann dans le chapitre pigment inorganique. On peut citer, parmi les pigments minéraux utiles dans la présente invention, les oxydes de fer ou de chrome, le violet de manganèse, le bleu outremer, l'hydrate de chrome, le bleu ferrique et l'oxyde de titane.

Le pigment peut-être un pigment organique. Par pigment organique, on entend tout pigment qui répond à la définition de l'encyclopédie Ullmann dans le chapitre pigment organique. Le pigment organique peut notamment être choisi parmi les composés nitroso, nitro, azo, xanthène, quinoléine, anthraquinone, phtalocyanine, de type complexe métallique, isoindolinone, isoindoline, quinacridone, périnone, pérylène, dicétopyrrolopyrrole, thioindigo, dioxazine, triphénylméthane, quinophtalone.

En particulier, les pigments organiques blancs ou colorés peuvent être choisis parmi le carmin, le noir de carbone, le noir d'aniline, le jaune azo, la quinacridone, le bleu de phtalocyanine, le rouge sorgho, les pigments bleus codifiés dans le Color Index sous les références Cl 42090, 69800, 69825, 73000, 74100, 74160, les pigments jaunes codifiés dans le Color Index sous les références Cl 11680, 11710, 15985, 19140, 20040, 21100, 21108, 47000, 47005, les pigments verts codifiés dans le Color Index sous les références Cl 61565, 61570, 74260, les pigments oranges codifiés dans le Color Index sous les réfénces CI 11725, 15510, 45370, 71105, les pigments rouges codifiés dans le Color Index sous les références CI 12085, 12120, 12370, 12420, 12490, 14700, 15525, 15580, 15620, 15630, 15800, 15850, 15865, 15880, 17200, 26100, 45380, 45410, 58000, 73360, 73915, 75470, les pigments obtenus par polymérisation oxydante de dérivés indoliques, phénoliques tels qu'ils sont décrits dans le brevet FR 2 679 771.

Les pigments conformes à l'invention peuvent aussi être sous forme de pigments composites tels qu'ils sont décrits dans le brevet EP 1 184 426. Ces pigments composites peuvent être composés notamment de particules comportant un noyau inorganique, au moins un liant assurant la fixation des pigments organiques sur le noyau, et au moins un pigment organique recouvrant au moins partiellement le noyau.

Le pigment organique peut aussi être une laque. Par laque, on entend les colorants adsorbés sur des particules insolubles, l'ensemble ainsi obtenu restant insoluble lors de l'utilisation.

Les substrats inorganiques sur lesquels sont adsorbés les colorants sont par exemple l'alumine, la silice, le borosilicate de calcium et de sodium ou le borosilicate de calcium et d'aluminium, et l'aluminium.

Parmi les colorants, on peut citer le carmin de cochenille. On peut également citer les colorants connus sous les dénominations suivantes : D & C Red 21 (CI 45 380), D & C Orange 5 (CI 45 370), D & C Red 27 (CI 45 410), D & C Orange 10 (CI 45 425), D & C Red 3 (CI 45 430), D & C Red 4 (CI 15 510), D & C Red 33 (CI 17 200), D & C Yellow 5 (CI 19 140), D & C Yellow 6 (CI 15 985), D & C Green (CI 61 570), D & C Yellow 1 O (CI 77 002), D & C Green 3 (CI 42 053), D & C Blue 1 (CI 42 090).

A titre d'exemples de laques, on peut citer le produit connu sous la dénomination suivante : D & C Red 7 (CI 15 850:1).

Le pigment peut aussi être un pigment à effets spéciaux. Par pigments à effets spéciaux, on entend les pigments qui créent d'une manière générale une apparence colorée (caractérisée par une certaine nuance, une certaine vivacité et une certaine clarté) non uniforme et changeante en fonction des conditions d'observation (lumière, température, angles d'observation...). Ils s'opposent par-là même aux pigments colorés qui procurent une teinte uniforme opaque, semi-transparente ou transparente classique.

Il existe plusieurs types de pigments à effets spéciaux, ceux à faible indice de réfraction tels que les pigments fluorescents, photochromes ou thermochromes, et ceux à plus fort indice de réfraction tels que les nacres ou les paillettes.

A titre d'exemples de pigments à effets spéciaux, on peut citer les pigments nacrés tels que le mica titane recouvert avec un oxyde de fer, le mica recouvert avec un oxyde de fer, le mica recouvert d'oxychlorure de bismuth, le mica titane recouvert avec de l'oxyde de chrome, le mica titane recouvert avec un colorant organique notamment du type précité ainsi que les pigments nacrés à base d'oxychlorure de bismuth. Il peut également s'agir de particules de mica à la surface desquelles sont superposées au moins deux couches successives d'oxydes métalliques et/ou de matières colorantes organiques.

Les nacres peuvent plus particulièrement posséder une couleur ou un reflet jaune, rose, rouge, bronze, orangé, brun, or et/ou cuivré.

A titre illustratif des nacres pouvant être mises en oeuvre dans le cadre de la présente invention, on peut notamment citer les nacres de couleur or notamment commercialisées par la société ENGELHARD sous le nom Gold 222C (Cloisonne), Sparkle gold (Timica), Gold 4504 (Chromalite) et Monarch gold 233X (Cloisonne) ; les nacres bronzes notamment commercialisées par la société MERCK sous la dénomination Bronze fine (17384) (Colorona) et Bronze (17353) (Colorona), par la société Eckart sous la dénomination Prestige Bronze et par la société ENGELHARD sous la dénomination Super bronze (Cloisonne) ; les nacres oranges notamment commercialisées par la société ENGELHARD sous la dénomination Orange 363C (Cloisonne) et Orange MCR 101 (Cosmica) et par la société MERCK sous la dénomination Passion orange (Colorona) et Matte orange (17449) (Microna) ; les nacres de teinte brune notamment commercialisées par la société ENGELHARD sous la dénomination Nu-antique copper 340XB (Cloisonne) et Brown CL4509 (Chromalite) ; les nacres à reflet cuivre notamment commercialisées par la société ENGELHARD sous la dénomination Copper 340A (Timica) et par la société Eckart sous la dénomination Prestige Copper ; les nacres à reflet rouge notamment commercialisées par la société MERCK sous la dénomination Sienna fine (17386) (Colorona) ; les nacres à reflet jaune notamment commercialisées par la société ENGELHARD sous la dénomination Yellow (4502) (Chromalite) ; les nacres de teinte rouge à reflet or notamment commercialisées par la société ENGELHARD sous la dénomination Sunstone G012 (Gemtone) ; les nacres noires à reflet or notamment commercialisées par la société ENGELHARD sous la dénomination Nu antique bronze 240 AB (Timica), les nacres bleues notamment commercialisées par la société MERCK sous la dénomination Matte blue (17433) (Microna), Dark Blue (117324) (Colorona), les nacres blanches à reflet argenté notamment commercialisées par la société MERCK sous la dénomination Xirona Silver et les nacres orangées rosées vert doré notamment commercialisées par la société MERCK sous la dénomination Indian summer (Xirona) et leurs mélanges.

En plus des nacres sur un support mica, on peut envisager les pigments multicouches basés sur des substrats synthétiques comme l'alumine, la silice, le borosilicate de calcium et de sodium ou le borosilicate de calcium et d'aluminium, et l'aluminium.

On peut également citer les pigments à effet interférentiel non fixés sur un substrat comme les cristaux liquides (Helicones HC de Wacker), les paillettes holographiques interférentielles (Geometric Pigments ou Spectra f/x de Spectratek). Les pigments à effets spéciaux comprennent aussi les pigments fluorescents, que ce soit les substances fluorescentes à la lumière du jour ou qui produisent une fluorescence ultraviolette, les pigments phosphorescents, les pigments photochromiques, les pigments thermochromiques et les quantum dots, commercialisés par exemple par la société Quantum Dots Corporation.

La variété des pigments qui peuvent être utilisés dans la présente invention permet d'obtenir une riche palette de couleurs, ainsi que des effets optiques particuliers tels que des effets métalliques, interférentiels.

La taille du pigment utilisé dans la composition cosmétique contenant le ou les polymères filmogènes hydrophobes est généralement comprise entre 10 nm et 200 µm, de préférence entre 20 nm et 80 µm, et plus préférentiellement entre 30 nm et 50 µm.

Les pigments peuvent être dispersés dans le produit grâce à un agent dispersant.

L'agent dispersant sert à protéger les particules dispersées contre leur agglomération ou floculation. Cet agent dispersant peut être un tensioactif, un oligomère, un polymère ou un mélange de plusieurs d'entre eux, portant une ou des fonctionnalités ayant une affinité forte pour la surface des particules à disperser. En particulier, ils peuvent s'accrocher physiquement ou chimiquement à la surface des pigments. Ces dispersants présentent, en outre, au moins un groupe fonctionnel compatible ou soluble dans le milieu continu. En particulier, on utilise les esters de l'acide hydroxy-12 stéarique en particulier et d'acide gras en C8 à C20 et de polyol comme le glycérol, la diglycérine, tel que le stéarate d'acide poly(12-hydroxystéarique) de poids moléculaire d'environ 750g/mole tel que celui vendu sous le nom de Solsperse 21 000 par la société Avecia, le polygycéryl-2 dipolyhydroxystéarate (nom CTFA) vendu sous la référence Dehymyls PGPH par la société Henkel ou encore l'acide polyhydroxystéarique tel que celui vendu sous la référence Arlacel P100 par la société Uniqema et leurs mélanges.

Comme autre dispersant utilisable dans les compositions de l'invention, on peut citer les dérivés ammonium quaternaire d'acides gras polycondensés comme le Solsperse 17 000 vendu par la société Avecia, les mélanges de poly diméthylsiloxane/oxypropylène tels que ceux vendus par la société Dow Corning sous les références DC2-5185, DC2-5225 C.

Les pigments utilisés dans les compositions contenant le ou les polymères filmogènes hydrophobes peuvent être traités en surface par un agent organique.

Ainsi les pigments préalablement traités en surface utiles dans le cadre de l'invention sont des pigments qui ont subi totalement ou partiellement un traitement de surface de nature chimique, électronique, électro-chimique, mécano-chimique ou mécanique, avec un agent organique tel que ceux qui sont décrits notamment dans Cosmetics and Toiletries, Février 1990, Vol. 105, p. 53-64 avant d'être dispersés dans la composition conforme à l'invention. Ces agents organiques peuvent être par exemple choisis parmi les acides aminés ; les cires, par exemple la cire de carnauba et la cire d'abeille ; les acides gras, les alcools gras et leurs dérivés, tels que l'acide stéarique, l'acide hydroxystéarique, l'alcool stéarylique, l'alcool hydroxystéarylique, l'acide laurique et leurs dérivés ; les tensio-actifs anioniques ; les lécithines ; les sels de sodium, potassium, magnésium, fer, titane, zinc ou aluminium d'acides gras, par exemple le stéarate ou laurate d'aluminium ; les alcoxydes métalliques ; les polysaccharides, par exemple le chitosane, la cellulose et ses dérivés ; le polyéthylène ; les polymères (méth)acryliques, par exemple les polyméthylmethacrylates ; les polymères et copolymères contenant des motifs acrylates ; les protéines ; les alcanoamines ; les composés siliconés, par exemple les silicones, les polydiméthylsiloxanes, les alcoxysilanes, les alkylsilanes, les siloxy-silicates ; les composés organiques fluorés, par exemple les perfluoroalkyle éthers ; les composés fluoro-siliconés.

Les pigments traités en surface utiles dans les compositions cosmétiques contenant le ou les polymères filmogènes hydrophobes peuvent aussi avoir été traités par un mélange de ces composés et / ou avoir subi plusieurs traitements de surface.

Les pigments traités en surface utiles dans le cadre de la présente invention peuvent être préparés selon des techniques de traitement de surface bien connues de l'homme de l'art ou trouvés tels quels dans le commerce.

De préférence, les pigments traités en surface sont recouverts par une couche organique.

L'agent organique avec lequel sont traités les pigments peut être déposé sur les pigments par évaporation de solvant, réaction chimique entre les molécules de l'agent de surface ou création d'une liaison covalente entre l'agent de surface et les pigments.

Le traitement en surface peut ainsi être réalisé par exemple par réaction chimique d'un agent de surface avec la surface des pigments et création d'une liaison covalente entre l'agent de surface et les pigments ou les charges. Cette méthode est notamment décrite dans le brevet US 4 578 266.

De préférence, on utilisera un agent organique lié aux pigments de manière covalente.

L'agent pour le traitement de surface peut représenter de 0,1 à 50 % en poids du poids total des pigments traités en surface, de préférence de 0,5 à 30 % en poids, et encore plus préférentiellement de 1 à 10 % en poids.

De préférence, les traitements en surface des pigments sont choisis parmi les traitements suivants :
- un traitement PEG-Silicone comme le traitement de surface AQ commercialisé par LCW ;
- un traitement Chitosane comme le traitement de surface CTS commercialisé par LCW ;
- un traitement Triéthoxycaprylylsilane comme le traitement de surface AS commercialisé par LCW ;
- un traitement Méthicone comme le traitement de surface SI commercialisé par LCW ;
- un traitement Diméthicone comme le traitement de surface Covasil 3.05 commercialisé par LCW ;
- un traitement Diméthicone / Triméthylsiloxysilicate comme le traitement de surface Covasil 4.05 commercialisé par LCW ;
- un traitement Lauroyl Lysine comme le traitement de surface LL commercialisé par LCW ;
- un traitement Lauroyl Lysine Diméthicone comme le traitement de surface LL / SI commercialisé par LCW ;
- un traitement Myristate de Magnésium comme le traitement de surface MM commercialisé par LCW ;
- un traitement Dimyristate d'Aluminium comme le traitement de surface MI commercialisé par Miyoshi ;
- un traitement Perfluoropolyméthylisopropyl éther comme le traitement de surface FHC commercialisé par LCW ;
- un traitement Isostéaryl Sébacate comme le traitement de surface HS commercialisé par Miyoshi ;
- un traitement Disodium Stéaroyl Glutamate comme le traitement de surface NAI commercialisé par Miyoshi ;
- un traitement Diméthicone / Disodium Stéaroyl Glutamate comme le traitement de surface SA / NAI commercialisé par Miyoshi ;
- un traitement Phosphate de Perfluoroalkyle comme le traitement de surface PF commercialisé par Daito ;
- un traitement Copolymère acrylate / Diméthicone et Phosphate de Perfluoalkyle comme le traitement de surface FSA commercialisé par Daito ;
- un traitement Polyméthylhydrogène siloxane / Phosphate de Perfluoroalkyle comme le traitement de surface FS01 commercialisé par Daito ;
- un traitement Lauryl Lysine / Aluminium Tristéarate comme le traitement de surface LL-StAI commercialisé par Daito ;
- un traitement Octyltriéthylsilane comme le traitement de surface OTS commercialisé par Daito ;
- un traitement Octyltriéthylsilane / Phosphate de Perfluoroalkyle comme le traitement de surface FOTS commercialisé par Daito ;
- un traitement Copolymère Acrylate / Diméthicone comme le traitement de surface ASC commercialisé par Daito ;
- un traitement Isopropyl Titanium Triisostéarate comme le traitement de surface ITT commercialisé par Daito ;
- un traitement Cellulose Microcrystalline et Carboxyméthyl Cellulose comme le traitement de surface AC commercialisé par Daito ;
- un traitement Cellulose comme le traitement de surface C2 commercialisé par Daito ;
- un traitement copolymère Acrylate comme le traitement de surface APD commercialisé par Daito ;
- un traitement Phosphate de Perfluoroalkyle / Isopropyl Titanium Triisostéarate comme le traitement de surface PF + ITT commercialisé par Daito.

La composition contenant le ou les polymères filmogènes hydrophobes conforme à la présente invention peut de plus comprendre un ou plusieurs pigments non traités en surface.

De préférence le pigment est une nacre.

Le ou les pigments peuvent être compris dans une teneur allant de 0,5 jusqu'à 40% en poids, de préférence allant de 1 à 20 % en poids, par rapport au poids total de la composition selon l'invention.

Selon l'invention, la composition prête à l'emploi et la composition cosmétique comprenant le ou les polymères filmogènes hydrophobes et le ou les pigments appliquées sur les cheveux contiennent un ou plusieurs solvants volatils. Dans le cadre de l'invention, on entend par solvant volatil, un composé liquide à la température ambiante (20°C) et à la pression atmosphérique présentant une pression de vapeur à 20°C supérieure à 0,1 mmHg et de préférence comprise entre 0,1 et 300 mmHg, encore plus préférentiellement entre 0,5 et 200 mmHg.

Ce solvant volatil peut être de l'eau, un solvant organique non siliconé, un solvant organique siliconé ou leurs mélanges. A titre de solvant organique non siliconé volatil, on peut citer :
- les alcanols volatils en C1-C4 tels que l'éthanol, l'isopropanol;
- les alcanes volatils en C5-C7 tels que le n-pentane, l'hexane, le cyclopentane, le 2,3-diméthylbutane, le 2,2-diméthylbutane, le 2-méthylpentane, le 3-méthylpentane ;
- les esters d'acides en C1-C20 liquides et d'alcools en C1-C8 volatils tels que l'acétate de méthyle, l'acétate de n-butyle, l'acétate d'éthyle, l'acétate de propyle, l'acétate d'isopentyle, le 3-éthoxypopionate d'éthyle;
- les cétones liquides à température ambiante et volatiles telles que la méthyléthylcétone, la méthylisobutylcétone, la diisobutylcétone, l'isophorone, la cyclohexanone, l'acétone ;
- les éthers volatils tels que le diméthoxyméthane, le diéthoxyéthane, le diéthyléther ;
- les éthers de glycol volatils comme le 2-butoxyéthanol, le butyle diglycol, le monoméhyléther de diéthylène glycol, le n-butyléther de propylène glycol, l'acétate de monométhyl éther de propylène glycol.
- les huiles hydrocarbonées volatiles telles que les huiles volatiles hydrocarbonées ayant de 8 à 16 atomes de carbone et leurs mélanges, et notamment les alcanes ramifiés en C8-C16 comme les iso-alcanes (appelées aussi isoparaffines) en C8-C16, l'isododécane, l'isodécane, et par exemple les huiles vendues sous les noms commerciaux d'Isopars ou de Permetyls, et leurs mélanges. On peut aussi citer les neopentanoate d'isohexyle ou d'isodecyle.
- les perfluoroalcanes volatils en C4-C10 tels que dodecafluoropentane, le tetradecafluorohexane, le decafluoropentane
- les perfluorocycloalkyles volatils tels que le perfluorométhylcyclopentane, le 1,3-perfluorodiméthylcyclohexane et le perfluorodecaline, vendus respectivement sous les dénominations de "Flutec PC1®", "Flutec PC3®" et "Flutec PC6®" par la Société F2 Chemicals, ainsi que le perfluorodiméthylcyclobutane et la perfluoromorpholine.
- les composés fluoroalkyles ou hétérofluoroalkyles volatils répondant à la formule suivante :

   CH₃-(CH₂)ₙ-[Z]ₜ-X-CF₃
dans laquelle t est 0 ou 1 ; n est 0, 1, 2 ou 3 ; X est un radical perfluoroalkyle divalent, linéaire ou ramifié, ayant de 2 à 5 atomes de carbone, et Z représente O, S, ou NR, R étant hydrogène, un radical - (CH2)n-CH3 ou -(CF2)m-CF3, m étant 2, 3, 4 ou 5.

Parmi les composés fluoroalkyles ou hétérofluoroalkyles volatils, on peut notamment citer le méthoxynonafluorobutane vendu sous la dénomination de "MSX 4518®", "HFE-7100®" par la Société 3M et l'éthoxynonafluorobutane vendu sous la dénomination de "HFE-7200®" par la Société 3M.

De préférence, le solvant est choisi de telle manière que son point d'ébullition soit inférieur à 200°C.

Selon un mode de réalisation particulier, le solvant organique non siliconé est choisi parmi l'éthanol, l'isopropanol, l'acétone, l'isododécane.

A titre de solvant siliconé volatil, on peut citer les composés siliconés à faible viscosité choisi parmi les silicones linéaires ou cycliques ayant de 2 à 7 atomes de silicium, ces silicones comportant éventuellement des groupes alkyle ou alkoxy ayant de 1 à 10 atomes de carbone, par exemple l'octaméthylcyclotétrasiloxane, le décaméthylcyclopentasiloxane, le dodécaméthylcyclohexasiloxane, l'heptaméthylhexyltri-siloxane, l'heptaméthyléthyltrisiloxane, l'octaméthyltrisiloxane, le décaméthyltétrasiloxane, et leurs mélanges. Selon un mode de réalisation particulier, le composé siliconé est choisi parmi le cyclopentadiméthylsiloxane, le dodécaméthylcyclohexasiloxane, l'octaméthyltrisiloxane et le décaméthyltétrasiloxane.

A titre d'exemple on peut citer la décaméthylcyclopentasiloxane commercialisée sous le nom DC-245 par la société Dow Corning, l'octaméthyltrisiloxane commercialisée sous le nom DC-200 Fluid 1 est par la société Dow Corning et le décaméthyltétrasiloxane commercialisée sous le nom DC-200 Fluid 1.5 est par la société Dow Corning

Le ou les solvants volatils peuvent être présents dans la composition cosmétique prête à l'emploi et dans la composition comprenant le ou les polymères filmogènes et le ou les pigments en une teneur allant de 0,1 % à 95 % en poids, par rapport au poids total de la composition, de préférence allant de 1 % à 90 % en poids, et préférentiellement allant de 5 % à 90 % en poids, par rapport au poids total de la composition.

La composition cosmétique prête à l'emploi et la composition comprenant le ou les polymères filmogènes, le ou les pigments et le ou les solvants volatils conformes à l'invention peuvent de plus contenir d'autres solvants organiques non volatils tels que :
- les alcools aromatiques non volatils tels que l'alcool benzylique, le phenoxyéthanol ;
- les esters d'acides en C1-C20 liquides et d'alcools en C1-C8 non volatils tels que le myristate d'isopropyle ;
- l'éthylène carbonate, le propylène carbonate; le butylène carbonate ;
- les polyols non volatils tels que le glycérol, l'éthylène glycol, le dipropylène glycol, le butylène glycol,
- les éthers de glycol non volatils comme le monoéthyléther de diéthylène glycol, le mono n-butyl éther de dipropylène glycol.
- les huiles hydrocarbonées non volatiles telles que l'isohexadécane.
- Les alcools gras liquides non volatils en C10-C30 tels que l'alcool oléique, les esters d'alcools gras en C10-C30 liquides tels que les benzoates d'alcool gras en C10-C30 et leurs mélanges ; l'huile de polybutène, l'isononanoate d'isononyle, le malate d'isostéaryle, le tétra-isostéarate de pentaérythrityle, le trimélate de tridécyle
- Les solvants perfluorés non volatils tels que le perfluoroperhydrophenanthrene, vendu sous la dénomination de "Flutec PC11®" par la Société F2 Chemicals.

La composition cosmétique prête à l'emploi et la composition comprenant le ou les polymères filmogènes hydrophobes, le ou les pigments et le ou les solvants volatils peuvent contenir d'autres espèces colorées ou colorantes que les pigments telle que des colorants directs hydrophiles ou hydrophobes ou des précurseurs de colorants.

Afin d'obtenir un meilleur étalement de la composition prête à l'emploi et de la composition comprenant le ou les polymères filmogènes hydrophobes, le ou les pigments et le ou les solvants volatils conformes à l'invention ainsi qu'un gainage amélioré, lesdites compositions selon l'invention peuvent également contenir un ou plusieurs polysiloxanes présentant une viscosité supérieure à 100 est, préférentiellement supérieure à 300 est. La viscosité de ces polysiloxanes peut être mesurée selon la norme ASTM D-445. De tels polysiloxanes peuvent être des huiles, des gommes ou des résines de silicone, les silicones réticulées.

Le ou les polysiloxanes pouvant être présents dans lesdites compositions selon l'invention sont différents des polymères filmogènes hydrophobes.

Par ailleurs, le ou les polysiloxanes pouvant être présents dans lesdites compositions selon l'invention sont également différents du solvant volatil siliconé.

A titre de polysiloxanes de viscosité supérieure à 100 est, on peut notamment citer les polydiméthylsiloxanes; les alkyldiméthicones; les polyphénylméthylsiloxanes tels que les phényldiméthicones, les phényltriméthicones, et les vinylméthylméthicones; ainsi que les silicones modifiées par des groupements aliphatiques et/ou aromatiques, éventuellement fluorés, ou par des groupements fonctionnels tels que des groupements hydroxyles, thiols et/ou amines.

De tels polysiloxanes peuvent choisis parmi les silicones de formule (V): 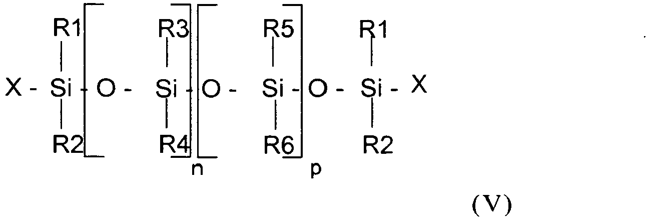 dans laquelle :
R₁, R₂, R₅ et R₆ sont, ensemble ou séparément, un radical alkyle ayant 1 à 6 atomes de carbone, R₃ et R₄ sont, ensemble ou séparément, un radical alkyle ayant de 1 à 6 atomes de carbone, un radical vinyle, un radical aryle, un radical amine, un radical hydroxyle, X est un radical alkyle ayant de 1 à 6 atomes de carbone, un radical hydroxyle, un radical vinyle, un radical amine, n et p étant des entiers choisis de manière à obtenir une viscosité supérieure à 300 est.

A titre d'exemple, on peut citer les polydiméthylsiloxanes suivantes :
∘ les substituants R₁ à R₆ et X représentent un groupement méthyle, comme celle vendue sous la dénomination Baysilicone TP 3898 par la société Général Electric, et celle vendue sous la dénomination AK 500000 par la société Waker,
∘ les substituants R₁ à R₆ et X représentent un groupement méthyle, p et n sont tels que le poids moléculaire est de 120 000 g/mol, comme celle vendue sous la dénomination Dow Corning 200 Fluid 60000 CS par la société Dow Corning.
∘ les substituants R₁ à R₆ et X représentent un groupement méthyle, p et n sont tels que le poids moléculaire est de 250 000 g/mol, comme celle vendue sous la dénomination Mirasil DM 500.000 par la société Rhodia et celle vendue sous la dénomination Dow Corning 200 Fluid 500.000 est par la société Dow Corning.
∘ les substituants R₁ à R₆ représentent un groupement méthyle, le groupement X représente un groupement hydroxy, n et p sont tels que le poids moléculaire du polymère soit de 600 000 g/mol, comme celle vendue sous la dénomination SGM 36 par la société Dow Corning.
∘ Les diméthicones du type (polydiméthylsiloxane)(méthylvinylsiloxane) telle que la SE63 commercialisée par GE BAYER Silicones, les copolymères poly(diméthylsiloxane)(diphényl)(méthylvinylsiloxane), et leurs mélanges.

Dans le cas où le polysiloxane comprend un groupement fluoré, on peut choisir les copolymères de structure suivante : 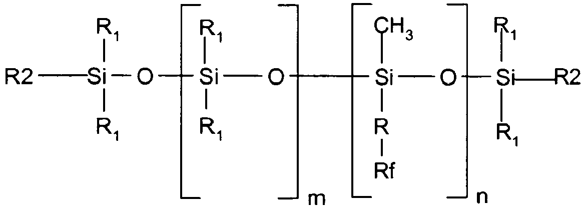 dans laquelle :
R représente un groupement divalent alkyle linéaire ou ramifié, ayant 1 à 6 atomes de carbone, de préférence un groupement divalent méthyle, éthyle, propyle ou butyle, Rf représente un radical fluoroalkyle, notamment un radical perfluoroalkyle, ayant 1 à 12 atomes de carbone, de préférence 1 à 9 atomes de carbone, R₁ représente, indépendamment l'un de l'autre, un radical alkyle en C₁-C₂₀, un radical hydroxyle, un radical phényle, R₂ représente R₁ ou R_{f}
   - m est choisi de 0 à 500, de préférence de 0 à 200, et n est choisi de 1 à 1000, de préférence de 1 à 500.

De préférence, les groupements R1 sont identiques et représentent un radical méthyle.

De tels polysiloxanes sont notamment ceux commercialisés par la société Shin Etsu sous les dénominations 'FL-5', 'FL-10', 'X22-821' et 'X22-822' ou encore 'FL-100', par la société Dow Corning sous le nom FS-1265 Fluid, par la société Phoenix Chemical sous la gamme Pecosil FS sous les dénominations Pecosil FSL-150, Pecosil FSL-300, Pecosil FSH-150, Pecosil FSH-300, Pecosil FSU-150, Pecosil FSU-300.

La masse moléculaire en poids du ou des polysiloxane peut être comprise entre 1000 et 1 500 000 g/mol, notamment entre 20 000 et 1 000 000 g/mol.

Le polysiloxane peut être sous forme de résine. Par le terme « résine », on entend une structure tridimensionnelle réticulée ou non. A titre d'exemple de résine de polysiloxane, on peut citer les silsesquioxanes et les siloxysilicates.

Dans un mode de réalisation de l'invention, les polysiloxanes utiles dans la composition de l'invention sont solubles ou dispersables dans la composition de l'invention. Dans un mode de réalisation, la résine de silicone est solide à 25°C.

La composition prête à l'emploi ainsi que la composition contenant le ou les polymères filmogènes hydrophobes, le ou les pigments et le ou les solvants volatils conformes à l'invention peuvent aussi contenir une silicone réticulée telle qu'un organopolysiloxane élastomère réticulé, un composé siliconé de haut poids moléculaire présentant une structure tridimensionnelle, aux propriétés viscoélastiques d'un matériau solide souple. Ces organopolysiloxane peuvent ainsi se présenter sous forme sèche en poudre, ou sous forme gonflée, dans un solvant, le produit résultant étant généralement un gel. Ces produits peuvent également se présenter sous forme dispersée dans une solution aqueuse.

La synthèse de ces organopolysiloxane est décrite dans les brevets suivants :
- US 5266321 de Kobayashi Kose,
- US 4742142 de Toray Silicone,
- US 5654362 de Dow Corning Corp,
- la demande de brevet FR 2 864 784.

Les organopolysiloxanes élastomères utilisés dans lesdites compositions peuvent être partiellement ou totalement réticulés. Ils se présentent généralement sous forme de particules. En particulier, les particules d'organopolysiloxane élastomère ont une taille moyenne en nombre allant de 0,1 à 500 µm, de préférence de 3 à 200 µm et mieux de 3 à 50 µm. Ces particules peuvent avoir toute forme et par exemple être sphériques, plates ou amorphes.

L'organopolysiloxane réticulé obtenu peut être un composé non-émulsionnant ou un composé émulsionnant. Le terme "non émulsionnant" défini des organopolysiloxanes réticulés ne contenant pas de motifs polyoxyalkylène. Le terme "émulsionnant" signifie des composés organopoysiloxanes réticulés ayant au moins un motif polyoxyalkylène, notamment polyoxyéthylène ou polyoxypropylène.

Les particules d'organopolysiloxane réticulé peuvent être véhiculées sous forme de gel constitué d'un organopolysiloxane réticulé inclus dans au moins une huile hydrocarbonée et/ou une huile siliconée. Dans ces gels, les particules d'organopolysiloxane sont souvent des particules non-sphériques. Les particules d'organopolysiloxane réticulé peuvent également se présenter sous forme de poudre, notamment sous forme de poudre sphérique.

Des organopolysiloxanes réticulés non-émulsionnants sont notamment décrits dans les brevets US4970252, US4987169, US 5412004, US5654362, US5760116, dans la demande JP-A-61-194009.

Comme organopolysiloxanes réticulés non-émulsionnants, on peut utiliser ceux vendus sous les dénominations "KSG-6", "KSG-15", "KSG-16", "KSG-18", "KSG-31", "KSG-32", "KSG-33", "KSG-41", "KSG-42", "KSG-43", "KSG-44" « USG-103 » par la société Shin Etsu, "DC 9040", "DC9041", "DC 9509", "DC9505", "DC 9506" « DC 9045 » par la société Dow Corning, "GRANSIL" par la société Grant Industries, "SFE 839" par la société General Electric.

Avantageusement, les organopolysiloxanes réticulés émulsionnants comprennent les organopolysiloxanes modifiés polyoxyalkylène formé à partir de composés divinyliques, en particulier des polysiloxanes ayant au moins deux groupes vinyliques, réagissant avec des liaisons Si-H d'un polysiloxane. Des organopolysiloxanes réticulés émulsionnants sont notamment décrits dans les brevets US5236986, US5412004, US5837793, US5811487.

Comme organopolysiloxanes réticulés émulsionnants, on peut utiliser ceux commercialisés sous les dénominations "KSG-21", "KSG-20", "KSG-30", "X-226146" par la société Shin Etsu, et "DC9010", "DC9011" par la société Dow Corning.

Les particules d'organopolysiloxane réticulé élastomère peuvent se présenter également sous forme de poudre d'organopolysiloxane réticulé élastomère enrobée de résine de silicone, notamment de résine silsesquioxane, comme décrit par exemple dans le brevet US5538793.

De tels élastomères sont vendus sous les dénomination "KSP-100", "KSP-101", "KSP-102", "KSP-103", KSP-104", "KSP-105" par la société Shin Etsu.

Lorsqu'ils sont présents, la quantité de ces composés siliconés est généralement comprise entre 0,1 % et 30% en poids, notamment entre 0,1% et 20% en poids et préférentiellement entre 0.1 et 10% en poids.

La composition de pré-traitement et les compositions cosmétiques comprenant le ou les polymères filmogènes hydrophobes peuvent contenir un ou plusieurs agents épaississants choisis parmi les agents épaississants polymériques différents des polymères filmogènes hydrophobes de l'invention, les agents épaississants minéraux et leurs mélanges.

L'épaississant peut être minéral ou organique, polymère ou non polymère. L'épaississant peut être choisi pour épaissir une phase aqueuse ou une phase grasse de la composition selon les cas.

On entend par épaississant, un composé qui modifie la rhéologie du milieu dans lequel il est incorporé.

L'épaississant de milieu aqueux peut être choisi parmi :
- les argiles hydrophiles,
- la silice pyrogénée hydrophile,
- les épaississants cellulosiques hydrosolubles, tels que l'hydroxyethylcellulose, la méthylcellulose, l'hydroxypropylcellulose.

Parmi celles-ci on peut citer notamment les gommes vendues sous la dénomination Cellolize QP 4400 H par a société Amercol.
- les gommes de guar non ionique comprenant des groupements hydroxyalkyle en C₁-C₆. On peut mentionner à titre d'exemple, les groupements hydroxymethyle, hydroxypropyle et hydroxybutyle. De telles gommes de guar sont notamment vendue sous les dénominations commerciales JAGUAR HP8, JAGUAR HP60, JAGUAR HP120, JAGUAR HP105 par la société MEYHALL ou sous la dénomination GALACTASOL 40H4FD2 par la société AQUALON.
- les carraghénanes
- les gommes de caroube, de scléroglucane, de gellane, de rhamsan, de karaya
- les alginates, les maltodextrines, l'amidon et ses dérivés, l'acide hyaluronique et ses sels,
- les polymères poly(métha)crylates de glycéryle vendus sous les dénominations de "Hispagel" ou "Lubragel" par les Sociétés Hispano Quimica ou Guardian,
- l'alcool polyvinylique,
- les polymères et les copolymères réticulés d'acrylamide, tels que ceux vendus sous les dénominations de "PAS 5161" ou "Bozepol C" par la Société Hoechst, de "Sepigel 305" par la Société Seppic par la Société Allied Colloïd, ou encore
- les homopolymères réticulés de chlorure de méthacryloyloxyéthyltriméthylammonium vendus sous la dénomination de "Salcare SC95" par la Société Allied Colloïd.
- les polymères associatifs et notamment les polyuréthanes associatifs.

De tels épaississants sont notamment décrits dans les demandes EP-A-1400234, dont le contenu est incorporé à titre de référence.

L'épaississant de milieu huileux peut être choisi parmi
- les argiles organophiles,
- les silices pyrogénées hydrophobes
- les gommes de guar alkylées (avec groupe alkyle en C1-C6), telles que celles décrites dans EP-A-708114 ;
- les polymères gélifiant d'huile comme les polymères tribloc ou en étoile résultant de la polymérisation ou copolymérisation d'au moins monomère à groupe éthylénique, comme les polymères vendus sous la dénomination Kraton ;
- les polymères de masse moléculaire moyenne en poids inférieure à 100 000, comportant a) un squelette polymérique ayant des motifs de répétition hydrocarbonés pourvus d'au moins un hétéroatome, et éventuellement b) au moins une chaîne grasse pendante et/ou au moins une chaîne grasse terminale éventuellement fonctionnalisées, ayant de 6 à 120 atomes de carbone et étant liées à ces motifs hydrocarbonés, telles que décrites dans les demandes WO-A-02/056847, WO-A-02/47619 dont le contenu est incorporé à titre de référence ; en particulier les résines de polyamides (notamment comprenant des groupes alkyles ayant de 12 à 22 atomes de carbone) telles que celles décrites dans US-A-5783657 dont le contenu est incorporé à titre de référence.
- les résines de polyamides siliconées telles que décrites dans la demande EP-A-1266647 , dans la demande de brevet français déposée sous le n° 0216039 dont le contenu est incorporé à titre de référence.

De tels épaississants sont notamment décrits dans les demandes EP-A-1400234, dont le contenu est incorporé à titre de référence.

L'épaississant peut être un agent gélifiant organique, c'est-à-dire un agent comprenant au moins un composé organique. Les organogélateurs peuvent être choisis parmi ceux décrits dans la demande WO-A-03/105788 dont le contenu est incorporé à titre de référence.

Plus précisément, l'agent épaississant polymérique est un polymère amorphe formé par polymérisation d'une oléfine. L'oléfine peut être notamment un monomère à insaturation éthylénique élastomérique.

Comme exemple d'oléfine, on peut citer les monomères de carbure éthylénique, ayant notamment une ou deux insaturations éthylénique, ayant de 2 à 5 atomes de carbone tels que l'éthylène, le propylène, le butadiène, l'isoprène.

L'agent épaississant polymérique est capable d'épaissir ou de gélifier la composition. Par polymère amorphe, on entend un polymère qui n'a pas de forme cristalline. L'agent épaississant polymérique peut être également filmogène.

L'agent épaississant polymérique peut être notamment un copolymère dibloc, tribloc, multibloc, radial, étoile, ou leurs mélanges.

De tels agents épaississants polymériques sont décrits dans la demande US-A-2002/005562 et dans le brevet US-A-5 221 534

Avantageusement, l'agent épaississant polymérique est un copolymère bloc amorphe de styrène et d'oléfine.

L'agent épaississant polymérique est de préférence hydrogéné pour réduire les insaturations éthyléniques résiduelles après la polymérisation des monomères.

En particulier, l'agent épaississant polymérique est un copolymère, éventuellement hydrogéné, à blocs styrène et à blocs éthylène/alkylène en C₃-C₄.

Comme copolymère dibloc, de préférence hydrogéné, on peut citer les copolymères de styrène-éthylène/propylène, les copolymères de styrène-éthylène/butadiène. Des polymères diblocs sont notamment vendus sous la dénomination Kraton® G1701E par la société Kraton Polymers.

Comme copolymère tribloc, de préférence hydrogéné, on peut citer les copolymères de styrène-éthylène/propylène-styrène, les copolymères de styrène-éthylène/butadiène-styrène, les copolymères de styrène-isoprène-styrène, les copolymères de styrène-butadiène-styrène. Des polymères triblocs sont notamment vendus sous les dénominations Kraton® G1650, Kraton® G1652, Kraton® D1101, Kraton® D1102, Kraton® D1160 par la société Kraton Polymers.

On peut également utiliser un mélange de copolymère hydrogéné tribloc styrène-butylène/éthylène-styrène et de polymère étoile hydrogéné éthylène-propylène-styrène, un tel mélange étant notamment dans l'isododécane. De tels mélanges sont par exemple vendus par la société PENRECO sous les dénominations commerciales VERSAGEL® M5960 et VERSAGEL® M5670.

Avantageusement, on utilise comme agent épaississant polymérique un copolymère dibloc tel que ceux décrits précédemment, en particulier un copolymère dibloc de styrène-éthylène/propylène.

Plus précisément, les argiles organophiles sont des argiles modifiées par des composés chimiques rendant l'argile apte à gonfler.

Les argiles sont des produits déjà bien connus en soi, qui sont décrits par exemple dans l'ouvrage "Minéralogie des argiles, S. Caillère, S. Hénin, M. Rautureau, 2ème édition 1982, Masson", dont l'enseignement est ici inclus à titre de référence.

Les argiles sont des silicates contenant un cation pouvant être choisi parmi les cations de calcium, de magnésium, d'aluminium, de sodium, de potassium, de lithium et leurs mélanges.

A titre d'exemples de tels produits, on peut citer les argiles de la famille des smectites telles que les montmorillonites, les hectorites, les bentonites, les beidellites, les saponites, ainsi que de la famille des vermiculites, de la stévensite, des chlorites.

Ces argiles peuvent être d'origine naturelle ou synthétique. De préférence, on utilise les argiles qui sont cosmétiquement compatibles et acceptables avec les matières kératiniques.

L'argile organophile peut être choisie parmi la montmorrilonite, la bentonite, l'hectorite, l'attapulgite, la sépiolite, et leurs mélanges. L'argile est de préférence une bentonite ou une hectorite.

Ces argiles peuvent être modifiées avec un composé chimique choisi parmi les amines quaternaires, les amines tertiaires, les acétates aminés, les imidazolines, les savons aminés, les sulfates gras, les alkyl aryl sulfonates, les oxides amines, et leurs mélanges.

Comme argiles organophiles, on peut citer les quaternium-18 bentonites telles que celles vendues sous les dénominations Bentone 3, Bentone 38, Bentone 38V par la société Rhéox, Tixogel VP par la société United catalyst, Claytone 34, Claytone 40, Claytone XL par la société Southern Clay; les stéaralkonium bentonites telles que celles vendues sous les dénominations Bentone 27 par la société Rheox, Tixogel LG par la société United Catalyst, Claytone AF, Claytone APA par la société Southern Clay ; les quaternium-18/benzalkonium bentonite telles que celles vendues sous les dénominations Claytone HT, Claytone PS par la société Southern Clay.

Les silices pyrogénées peuvent être obtenues par hydrolyse à haute température d'un composé volatil du silicium dans une flamme oxhydrique, produisant une silice finement divisée. Ce procédé permet notamment d'obtenir des silices hydrophiles qui présentent un nombre important de groupements silanol à leurs surfaces. De telles silices hydrophiles sont par exemple commercialisées sous les dénominations "AEROSIL 130®", "AEROSIL 200®", "AEROSIL 255®", "AEROSIL 300®", "AEROSIL 380®" par la société Degussa, "CAB-O-SIL HS-5®", "CAB-O-SIL EH-5®", "CAB-O-SIL LM-130®", "CAB-O-SIL MS-55®", "CAB-O-SIL M-5®" par la société Cabot.

Il est possible de modifier chimiquement la surface de ladite silice, par réaction chimique générant une diminution du nombre de groupes silanol. On peut notamment substituer des groupes silanol par des groupements hydrophobes : on obtient alors une silice hydrophobe.

Les groupements hydrophobes peuvent être :
- des groupements triméthylsiloxyl, qui sont notamment obtenus par traitement de silice pyrogénée en présence de l'hexaméthyldisilazane. Des silices ainsi traitées sont dénommées "Silica silylate" selon le CTFA (6ème édition, 1995). Elles sont par exemple commercialisées sous les références "AEROSIL R812®" par la société Degussa, "CAB-O-SIL TS-530®" par la société Cabot.
- des groupements diméthylsilyloxyl ou polydiméthylsiloxane, qui sont notamment obtenus par traitement de silice pyrogénée en présence de polydiméthylsiloxane ou du diméthyldichlorosilane. Des silices ainsi traitées sont dénomées "Silica diméthyl silylate" selon le CTFA (6ème édition, 1995). Elles sont par exemple commercialisées sous les références "AEROSIL R972®", "AEROSIL R974®" par la société Degussa, "CAB-O-SIL TS-610®", "CAB-O-SIL TS-720®" par la société Cabot.

La silice pyrogénée présente de préférence une taille de particules pouvant être nanométrique à micrométrique, par exemple allant d'environ de 5 à 200 nm.

De préférence, on utilise comme agent épaississant minéral une hectorite ou une bentonite organomodifiée.

Le ou les agents épaississants sont présents dans la composition en une teneur totale allant de 0,1 à 10% en poids par rapport au poids total de la composition comprenant le ou les polymères filmogènes hydrophobes, de préférence allant de 0,5 à 7 % en poids, et plus préférentiellement allant de 0,5 à 5% en poids.

Par milieu cosmétiquement acceptable, on entend un milieu compatible avec les fibres kératiniques, telles que les cheveux.

Le milieu cosmétiquement acceptable de la composition de pré-traitement selon l'invention est constitué d'eau, d'un solvant cosmétiquement acceptable ou d'un mélange d'eau et d'un ou plusieurs solvants cosmétiquement acceptables. Ces solvants cosmétiquement acceptable peuvent être choisis parmi les alcools inférieurs en C₁-C₄, tels que l'éthanol, l'isopropanol, les polyols et éthers polyols comme le 2-butoxyéthanol, le propylène glycol, le monométhyléther de propylène glycol, le monoéthyléther et le monométhyléther du diéthylèneglycol, ainsi que les alcools aromatiques comme l'alcool benzylique ou le phénoxyéthanol, et leurs mélanges.

De préférence, le milieu cosmétiquement acceptable de la composition cosmétique de pré-traitement est constitué d'eau, d'éthanol ou d'un mélange constitué d'eau et d'éthanol.

Le ou les composés organiques du silicium sont solubles dans le milieu cosmétiquement acceptable et encore plus préférentiellement solubles à la concentration de 1%, mieux à la concentration de 2% et encore mieux à la concentration de 5% en poids dans l'eau à la température de 25°C±5°C et à la pression atmosphérique. Par soluble, on entend la formation d'une phase macroscopique unique.

La teneur du milieu cosmétiquement acceptable de la composition cosmétique de pré-traitement peut varier de 0,1% à 99% en poids, de préférence de 5% à 98% en poids, par rapport au poids total de la composition de pré-traitement.

Les compositions selon l'invention peuvent également contenir un ou plusieurs adjuvants cosmétiques choisis, par exemple, parmi des agents réducteurs, des corps gras, des adoucissants, des agents anti-mousse, des agents hydratants, des filtres UV, des colloïdes minéraux, des peptisants, des solubilisants, des parfums, des tensio-actifs anioniques, cationiques, non ioniques ou amphotères, des protéines, des vitamines, des propulseurs, les cires oxyéthylénées ou non, les paraffines, les acides gras en C10-C30 tels que l'acide stéarique, l'acide laurique, les amides gras en C10-C30 tel que le diéthanolamide laurique.

Les additifs ci dessus sont en général présents en quantité comprise pour chacun d'eux entre 0,01 et 20 % en poids par rapport au poids de la composition.

Bien entendu, l'homme de l'art veillera à choisir ce ou ces éventuels additifs de manière telle que les propriétés avantageuses attachées intrinsèquement à la formation du gainage conforme à l'invention ne soient pas, ou substantiellement pas, altérées.

Les compositions conformes à l'invention peuvent se présenter notamment sous forme de suspension, de dispersion, de solution, de gel, d'émulsion, notamment émulsion huile-dans-eau (H/E) ou eau-dans-huile (E/H), ou multiple (E/H/E ou polyol/H/E ou H/E/H), sous forme de crème, de mousse, de stick, de dispersion de vésicules notamment de lipides ioniques ou non, de lotion biphasé ou multiphase, de spray, de poudre, de pâte. La composition peut également se présenter sous forme de laque.

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

Un autre objet de l'invention est un procédé de traitement cosmétique des fibres kératiniques, telles que les cheveux, qui consiste à appliquer une quantité efficace de la composition prête à l'emploi telle que décrite ci-dessus, sur lesdites fibres.

Selon une variante, il est possible de mélanger extemporanément avant application sur les cheveux, le ou les composés organiques du silicium, éventuellement solubilisés dans un solvant volatil, avec une composition comprenant un ou plusieurs polymères filmogènes hydrophobes, un ou plusieurs pigments et un ou plusieurs solvants volatils.

La composition prête à l'emploi décrite ci-dessus peut être mise en oeuvre sur cheveux secs ou humides ainsi que sur tous types de cheveux clairs ou foncés, naturels ou colorés, permanentés, décolorés ou défrisés.

De préférence, la composition prête à l'emploi selon l'invention est appliquée sur cheveux humides.

Selon un mode de réalisation particulier du procédé de l'invention, les cheveux sont lavés avant application de la composition décrite ci-dessus.

L'application sur les cheveux de la composition prête à l'emploi peut être mise en oeuvre par exemple au moyen d'un peigne, d'un pinceau à l'aide d'une brosse ou aux doigts.

La présente invention porte également sur un procédé de traitement cosmétique des fibres kératiniques telles que les cheveux, qui consiste à appliquer une composition cosmétique de pré-traitement comprenant, dans un milieu cosmétiquement acceptable, un ou plusieurs composés organiques du silicium tels que définis ci-avant, puis à appliquer une composition cosmétique comprenant un ou plusieurs polymères filmogènes hydrophobes, un ou plusieurs pigments et un ou plusieurs solvants volatils tels que définis ci-avant.

Plus particulièrement, le procédé de traitement consiste à appliquer sur lesdites fibres kératiniques, une composition cosmétique de pré-traitement comprenant, dans un milieu cosmétiquement acceptable, un ou plusieurs composés organiques du silicium tels que définis ci-avant, à rincer ou non après un éventuel temps de pose et à sécher ou non, puis à appliquer une composition cosmétique comprenant un ou plusieurs polymères filmogènes hydrophobes, un ou plusieurs pigments et un ou plusieurs solvants volatils tels que définis ci-avant.

Les compositions cosmétiques décrites ci-dessus peuvent être mises en oeuvre sur tous types de cheveux, clairs ou foncés, naturels ou sur des cheveux ayant subi un traitement cosmétique tel qu'une permanente, une coloration, une décoloration ou un défrisage.

La composition cosmétique de pré-traitement telle que décrite ci-dessus peut être mise en oeuvre sur cheveux secs ou humides. De préférence, la composition cosmétique de pré-traitement est appliquée sur cheveux propres.

De préférence, le temps de pose entre la composition de pré-traitement et la composition cosmétique comprenant le ou les polymères filmogènes hydrophobes peut être compris entre quelques secondes et 60 minutes, de préférence entre 30 secondes et 15 minutes et encore plus préférentiellement entre 1 minute et 5 minutes.

La composition de pré-traitement peut être rincée ou non avant l'application de la composition comprenant le ou les polymères filmogènes hydrophobes.

De préférence, la composition de pré-traitement est rincée, c'est-à-dire que son application est suivie d'une étape de rinçage.

La composition cosmétique comprenant le ou les polymères filmogènes hydrophobes, le ou les pigments et le ou les solvants volatils peut être mise en oeuvre sur cheveux secs ou humides. Le choix de la présence de l'étape de séchage dépend du polymère hydrophobe utilisé dans la composition.

L'application sur les cheveux desdites compositions décrites ci-dessus peut être mise en oeuvre par exemple au moyen d'un peigne, d'un pinceau à l'aide d'une brosse ou aux doigts.

Selon un mode de réalisation particulier, l'application de la composition prête à l'emploi et de la composition cosmétique comprenant le ou les polymères filmogènes est ensuite suivie d'un séchage à une température supérieure à 40°C. Selon un mode de réalisation particulier, cette température est supérieure à 45°C. Selon un mode de réalisation particulier, cette température est supérieure à 45°C et inférieure à 220°C.

Le séchage peut être réalisé immédiatement après l'application ou après un temps de pose pouvant aller de 1 minute à 30 minutes.

De préférence les cheveux sont séchés, en plus d'un apport de chaleur, avec un flux d'air. Ce flux d'air pendant le séchage permet d'améliorer l'individualisation du gainage.

Durant le séchage, une action mécanique sur les mèches peut être exercée telle qu'un peignage, un brossage, le passage des doigts.

L'étape de séchage du procédé de l'invention peut être mise en oeuvre avec un casque, un sèche-cheveux, un fer à lisser, un climazon...

Lorsque l'étape de séchage est mise en oeuvre avec un casque ou un sèche-cheveux, la température du séchage est comprise entre 40 et 110 degrés, de préférence entre 50 et 90 degrés.

Lorsque l'étape de séchage est mise en oeuvre avec un fer à lisser, la température du séchage est comprise entre 110 et 220 degrés, de préférence entre 140 et 200 degrés.

Une fois le séchage terminé, un rinçage ou un shampooing terminal peut éventuellement être réalisé.

Les exemples suivants servent à illustrer la présente invention.

### I. EXEMPLES DE COMPOSITIONS PRÊTE À L'EMPLOI

### EXEMPLE 1

Les compositions suivantes sont réalisées :

| Composition | 1a | 1b | 1c | 1d |
|---|---|---|---|---|
| 3-Aminopropyltriethoxysilane Dow Corning Z-6011 Silane (*) | 2 g | - | - | - |
| 3-Aminopropylméthyldiéthoxysilane commercialisé par Fluka sous la référence 09309 | - | 2 g | - | - |
| N-(2-aminoéthyl)-3-aminopropylltriéthoxysilane commercialisé par ABCR sous la référence AB153226 | - | - | 2 g | - |
| Bis[3-triéthoxysilylpropyl]amine commercialisé par ABCR sous la référence SIB1824.5 | - | - | - | 2 g |
| BioPSA 7-4405 (BioPSA 7-4400 dilué à 40% dans l'isododécane) (*) | 20 g | 20 g | 20 g | 20 g |
| Mélange Polydimethylsiloxane alpha-omega dihydroxyle / Cyclopentadiméthylsiloxane (14,7 / 85,3) commercialisé sous le nom DC1501 Fluid (*) | 10 g | 10 g | 10 g | 10 g |
| Polyméthylsilsesquioxane commercialisé sous le nom Wacker Belsil PMS MK Powder par la société Wacker | 3 g | 3 g | 3 g | 3 g |
| Nacre mica enrobé d'oxyde de fer brun commercialisée par Eckart sous le nom Prestige Bronze | 10 g | 10 g | 10 g | 10 g |
| Ethanol | 8 g | 8 g | 8 g | 8 g |
| Isododécane | Qs 100 g | Qs 100 g | Qs 100 g | Qs 100 g |

| | | | | |
|---|---|---|---|---|
| (*) commercialisé par Dow Corning | | | | |

0,6g de la composition 1a, 1b, 1c ou 1d est appliqué sur une mèche de 1g de cheveux permanentes propres et humides. Après 2 minutes de pose, la mèche est séchée au sèche-cheveux à une température de 80°C pendant 2 minutes.

On obtient une mèche colorée dont les cheveux sont individualisés et dont la couleur est rémanente au shampooing.

### EXEMPLE 2

La composition suivante est réalisée :

| Composition | 5 | 5bis |
|---|---|---|
| 3-Aminopropyltriéthoxysilane commercialisé sous la reference Dow Corning Z-6011 Silane par Dow Corning | 2 g | - |
| Copolymère diméthylpolysiloxane/urée commercialisé sous la référence Wacker-Belsil® UD 60 par Wacker | 10 g | 10 g |
| Polydiméthylsiloxane commercialisée par Dow Corning sous la référence Dow Corning 200 Fluid 60000 cs | 5 g | 5 g |
| Nacre mica enrobé d'oxyde de fer brun commercialisée par Eckart sous le nom Prestige Bronze | 10 g | 10 g |
| Isopropanol | 40 g | 40 g |
| Cyclopentadiméthylsiloxane commercialisé par Dow Corning sous le nom de DC245 Fluid | Qs 100 g | Qs 100 g |

0.6g de la composition 2 est appliqué sur une mèche sèche de 1g de cheveux permanentés propres et humides. Après 2 minutes de pose, la mèche est séchée au sèche-cheveux à une température de 80°C pendant 2 minutes. On obtient une mèche colorée dont les cheveux sont individualisés et dont la couleur est rémanente au shampooing.

Lorsque la composition 5bis est appliquée dans les mêmes conditions, la tenue aux shampooings de la couleur est nettement moins bonne.

### EXEMPLE 3

La composition suivante est réalisée :

| Composition | 3 | 3bis |
|---|---|---|
| N-(2-aminoéthyl)-3-aminopropylltriéthoxysilane Commercialisé par ABCR sous la référence AB 153226 | 2 g | - |
| Copolymères acrylates/C12-22 alkylméthacrylate en dispersion à 48% dans l'eau commercialisé par Rohm and Haas sous la référence SOLTEX OPT | 20 g | 20 g |
| 7-3100 Gum Blend HIP Emulsion commercialisée par Dow Corning | 20 g | 20 g |
| Nacre mica enrobé d'oxyde de fer brun commercialisée par Eckart sous le nom Prestige Bronze | 10 g | 10 g |
| Acide lactique | pH 10 final | pH 10 final |
| Eau | Qs 100 g | Qs 100 g |

0,6g de la composition 3 est appliqué sur une mèche de 1g de cheveux permanentés propres et humides. Après 2 minutes de pose, la mèche est séchée au sèche-cheveux à une température de 80°C pendant 2 minutes. On obtient une mèche colorée dont les cheveux sont individualisés et dont la couleur est rémanente au shampooing.

Lorsque la composition 3bis est appliquée dans les mêmes conditions, la tenue aux shampooings de la couleur est nettement moins bonne.

### II. EXEMPLES DE COMPOSITIONS DE PRÉ-TRAITEMENT

### EXEMPLE 1

Les compositions suivantes sont réalisées :

**Compositions 1**

| Composition | 1a | 1b | 1c |
|---|---|---|---|
| 3-Aminopropyltriéthoxysilane Dow Corning Z-6011 Silane (*) | 10 g | - | - |
| 3-Aminopropylméthyldiéthoxysilane commercialisé par Fluka sous la référence 09309 | - | 10 g | - |
| N-(2-aminoéthyl)-3-aminopropyltriéthoxysilane commercialisé par ABCR sous la référence AB 153226 | - | - | 10 g |
| Acide lactique | pH 10 final | pH 10 final | pH 10 final |
| Eau | Qs 100g | Qs 100g | Qs 100g |

**Compositions 2 :**

| Composition | 2a | 2b | 2c | 2d |
|---|---|---|---|---|
| Bis[méthyldiéthoxysilylpropyl]amine Commercialisé par ABCR sous la référence SIB1620.0 | 2,5g | - | 2,5g | - |
| Bis[3-triéthoxysilylpropyl]amine Commercialisé par ABCR sous la référence SId1824.5 | - | 2,5g | - | 2,5g |
| Ethanol | 65g | 65g | Qs 100 g | Qs 100g |
| Acide lactique | pH 10 final | - | pH 10 final | - |
| Acide acétique | - | pH 5 final | - | pH 5 final |
| Eau | Qs 100g | Qs 100g | - | - |

**Composition 3 :**

| | |
|---|---|
| BioPSA 7-4405 (BioPSA 7-4400 dilué à 40% dans l'isododécane) (*) | 20 g |
| Mélange Polydiméthylsiloxane alpha-omega dihydroxyle / Cyclopentadimethylsiloxane (14,7 / 85,3) commercialisé sous 1e nom DC1501 Fluid (*) | 10 g |
| Polyméthylsilsesquioxane commercialisé sous le nom Wacker Belsil PMS MK Powder par la société Wacker | 3 g |
| Nacre mica enrobé d'oxyde de fer brun commercialisé par Eckart sous le nom Prestige Bronze | 10g |
| Distéardimonium hectorite (10%) et propylène carbonate (3%) dans l'isododécane commercialisé par Elementis sous le nom Bentone Gel ISD V | 15g |
| Isododécane | Qs 100 g |

| | |
|---|---|
| (*) commercialisé par Dow Corning | |

0,6g de la composition 1a, 1b, 1c, 2a, 2b, 2c ou 2d est appliqué sur une mèche de 1g de cheveux permanentés propres et humides. Après 5 minutes de pose, la mèche est rincée. 0,6g de la composition 3 est ensuite appliqué sur la mèche humide. Après 2 minutes de pose, la mèche est séchée au sèche-cheveux à une température de 80°C pendant 2 minutes. On obtient une mèche colorée dont les cheveux sont individualisés et dont la couleur est rémanente au shampooing.

### EXEMPLE 2

La composition suivante est réalisée :

**Composition 4**

| | |
|---|---|
| Copolymère diméthylpolysiloxane/urée commercialisé sous la référence Wacker-Belsil® UD 60 par Wacker | 10g |
| Polydiméthylsiloxane commercialisée par Dow Corning sous la référence Dow Corning 200 Fluid 60000 cs | 5 g |
| Nacre mica enrobé d'oxyde de fer brun commercialisée par Eckart sous le nom Prestige Bronze | 10g |
| Isopropanol | 40 g |
| Cyclopentadiméthylsiloxane commercialisé par Dow Corning sous le nom de DC245 Fluid | Qs 100 g |

0,6g de la composition 1a, 1b, 1c de l'exemple lest appliqué sur une mèche de 1g de cheveux permanentés propres et humides. Après 5 minutes de pose, la mèche est rincée puis séchée au sèche-cheveux. 0,6g de la composition 4 est ensuite appliqué sur la mèche sèche. Après 2 minutes de pose, la mèche est séchée au sèche-cheveux à une température de 80°C pendant 2 minutes. On obtient une mèche colorée dont les cheveux sont individualisés et dont la couleur est rémanente au shampooing.

Lorsque seule la composition 4 est appliquée sans le pré-traitement, la tenue aux shampooings de la couleur est nettement moins bonne.

### EXEMPLE 3

La composition suivante est réalisée :

**Composition 5**

| | |
|---|---|
| Copolymères acrylates/C12-22 alkylméthacrylate en dispersion à 48% dans l'eau commercialisé par Rohm and Haas sous la référence SOLTEX OPT | 20 g |
| 7-3100 Gum Blend HIP Emulsion commercialisée par Dow Corning | 20 g |
| Acide lactique | pH 10 final |
| Nacre mica enrobé d'oxyde de fer brun commercialisée par Eckart sous le nom Prestige Bronze | 10g |
| Eau | Qs 100 g |

0,6g de la composition 1a, 1b, 1c de l'exemple 1 est appliqué sur une mèche de 1g de cheveux permanentés propres et humides. Après 5 minutes de pose, la mèche est rincée puis séchée au sèche-cheveux. 0,6g de la composition 5 est ensuite appliqué sur la mèche sèche. Après 2 minutes de pose, la mèche est séchée au sèche-cheveux à une température de 80°C pendant 2 minutes. On obtient une mèche colorée dont les cheveux sont individualisés et dont la couleur est rémanente au shampooing.

Lorsque seule la composition 5 est appliquée sans le pré-traitement, la tenue aux shampooings de la couleur est nettement moins bonne.

## Revendications

1. Composition cosmétique pour 1e traitement des fibres kératiniques comprenant:
- un ou plusieurs composés organiques du silicium choisis parmi les silanes comprenant un, deux ou trois atomes de silicium, lesdits composés organiques du silicium comportant en outre une ou plusieurs fonctions chimiques basiques et un ou plusieurs groupes hydroxyles ou hydrolysables par molécule ;
- un ou plusieurs polymères filmogènes hydrophobes,
- un ou plusieurs pigments, et
- un ou plusieurs solvants volatils.

2. Composition cosmétique selon la revendication 1 dans laquelle les fonctions chimiques basiques du composé organique du silicium sont choisies parmi les aminés primaires, secondaires ou tertiaires.

3. Composition cosmétique selon 1a revendication 1 ou 2 dans laquelle les groupes hydrolysables sont choisis parmi les groupes alcoxy, aryloxy et halogène.

4. Composition cosmétique selon l'une quelconque des revendications 1 à 3 dans laquelle le ou les composés organiques du silicium sont choisis parmi les composés de formule (I) : 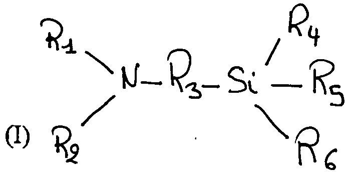 dans laquelle :
R₄ représente un halogène, un groupe OR' ou R'₁ ;
R₅ représente un halogène, un groupe OR" ou R'₂ ;
R₆ représente un halogène, un groupe OR'" ou R'₃ ;
R₁, R₂, R₃, R', R", R"', R'₁, R'₂, R'₃ représentent, indépendamment les uns des autres, un groupe hydrocarboné saturé ou insaturé, linéaire ou ramifié, portant éventuellement des groupes chimiques supplémentaires, R₁, R₂, R', R" et R"' pouvant en outre désigner l'hydrogène, et deux au moins des groupes R₄, R₅ et R₆ étant différents des groupes R'₁, R'₂ et R'₃.

5. Composition cosmétique selon la revendication 4 dans laquelle les groupes R₁, R₂, R', R'₁, R'₂, R'₃, R"et R'" sont choisis parmi les radicaux alkyle de C₁-C₁₂, aryle de C₅ à C₁₄, alkyl(C₁-C₈)aryle de C₅ à C₁₄, et aryl(C₅-C₁₄)alkyle de C₁ à C₈ et le groupe R₃ est choisi parmi les radicaux alkylène en C₁-C₁₂, éventuellement substitué par un groupement amino, arylène en C₅-C₁₄, alkylène(C₁-C₈)arylène en C₅-C₁₄, et arylène(C₅-C₁₄)alkylène en C₁-C₈.

6. Composition cosmétique selon l'une quelconque des revendications 1 à 3 dans laquelle 1e ou les composés organiques du silicium sont choisis parmi les composés de formule (II)
(R₂₁O)ₓ(R₂₂)_{y}Si-(B)ₚ- [NR₂₃-(B')_{p'}]_{q}-[NR'₂₃- (B")_{p"}]_{q'}-Si-(R'₂₂)_{y'}(OR'₂₁)_{x'} (II)
où
R₂₁, R₂₂, R'₂₁ et R'₂₂ représentent chacun indépendamment une chaîne hydrocarbonée saturée ou insaturée, linéaire ou ramifiée, contenant éventuellement un ou plusieurs hétéroatomes, éventuellement interrompue ou substituée par un ou plusieurs groupes choisis parmi les groupes éther, ester, amine, amide, carboxyle, hydroxyle et carbonyle,
x est un entier variant de 1 à 3,
y=3-x,
x' est un entier variant de 1 à 3,
y' = 3-x',
p = 0 ou 1,
p' = 0 ou 1,
p" = 0 ou 1,
q = 0 ou 1,
q' = 0 ou 1,
étant entendu que au moins q ou q' est différent de zéro, B, B' et B" représentent chacun indépendamment un radical divalent alkylène linéaire ou ramifié en C₁-C₂₀,
R₂₃ et R'₂₃ représentent chacun indépendamment un atome d'hydrogène ou une chaîne hydrocarbonée saturée ou insaturée, linéaire ou ramifiée, contenant éventuellement un ou plusieurs hétéroatomes, éventuellement interrompue ou substituée par un ou plusieurs groupes éther, ester d'alcool en C₁-C₂₀, amine, carboxyle, alcoxysilane, aryle en C₆-C₃₀, hydroxyle ou carbonyle, ou un cycle aromatique, hétérocyclique ou non, éventuellement substitué par un ou plusieurs groupes ester d'alcool en C₃-C₂₀, amine, amide, carboxyle, alcoxysilane, hydroxyle, carbonyle ou acyle.

7. Composition cosmétique selon l'une quelconque des revendications précédentes dans laquelle le composé organique du silicium est le 3-aminopropyltriéthoxysilane, le 3-aminopropylméthyldiéthoxysilane, le N-(2-aminoéthyl)-3-aminopropylltriéthoxysilane et le 3-(2-aminoethylamino)propyl-méthyldiéthoxysilane.

8. Composition cosmétique selon l'une quelconque des revendications précédentes dans laquelle le ou les polymères filmogènes hydrophobes sont choisis parmi les polyuréthanes ; polyuréthanes-acryliques ; les polyurées ; les polyurée-polyuréthanes ; les polyester-polyuréthanes ; les polyéther-polyuréthanes ; les polyesters ; les polyesters amides ; les polyester acryliques ; les polymères ou copolymères à base de polyvinylpyrrolidone ; les polymères ou copolymères acryliques et/ou vinyliques ; les polyacrylamides ; les polymères siliconés comprenant des parties acryliques ; les résines de silicone ; les silicones polyurée/polyuréthane ; les copolymères à base de résine de silicone et de diméthiconol ; les polymères fluorés ; les celluloses et leurs mélanges.

9. Composition cosmétique selon l'une quelconque des revendications précédentes dans laquelle le ou les solvants volatils sont choisis parmi l'eau et les solvants organiques choisis parmi l'éthanol, l'isopropanol, l'acétone, l'isododécane, décaméthylcyclopentasiloxane, l'octaméthyltrisiloxane et le décaméthyltétrasiloxane.

10. Composition cosmétique selon l'une quelconque des revendications précédentes dans laquelle le ou les pigments sont choisis parmi les pigments minéraux, les pigments organiques, les laques, les pigments à effets spéciaux tels que les nacres ou les paillettes, et leurs mélanges.

11. Composition cosmétique selon la revendication 10 dans laquelle le pigment est une nacre.

12. Procédé de traitement cosmétique des fibres kératiniques dans lequel on applique la composition cosmétique telle que définie à l'une quelconque des revendications précédentes, sur lesdites fibres, et on sèche la composition à une température supérieure à 40°C.

13. Utilisation d'une composition cosmétique selon l'une quelconque des revendications 1 à 11 pour la coloration des fibres kératiniques.

14. Utilisation sur les fibres kératiniques d'une composition cosmétique comprenant, dans un milieu cosmétiquement acceptable, un ou plusieurs composés organiques du silicium, choisis parmi les silanes comprenant un, deux ou trois atomes de silicium, lesdits composés organiques du silicium comportant en outre une ou plusieurs fonctions chimiques basiques et un ou plusieurs groupes hydroxyles ou hydrolysables par molécule en pré-traitement d'une composition cosmétique contenant un ou plusieurs polymères filmogènes hydrophobes, un ou plusieurs pigments et un ou plusieurs solvants volatils.

15. Procédé de traitement cosmétique des fibres kératiniques consistant à appliquer une composition cosmétique de pré-traitement comprenant, dans un milieu cosmétiquement acceptable, un ou plusieurs composés organiques du silicium tels que définis selon l'une quelconque des revendications 1 à 7, puis à appliquer une composition cosmétique comprenant, un ou plusieurs polymères filmogènes hydrophobes, un ou plusieurs pigments et un ou plusieurs solvants volatils.

## Patentansprüche

1. Kosmetische Zusammensetzung zur Behandlung von Keratinfasern, umfassend:
- eine oder mehrere organische Siliciumverbindungen, die aus Silanen mit einem, zwei oder drei Siliciumatomen ausgewählt sind, wobei die organischen Siliciumverbindungen außerdem eine oder mehrere basische chemische Funktionen und eine oder mehrere Hydroxylgruppen oder hydrolysierbare Gruppen pro Molekül umfassen;
- ein oder mehrere hydrophobe filmbildende Polymere,
- ein oder mehrere Pigmente und
- ein oder mehrere flüchtige Lösungsmittel.

2. Kosmetische Zusammensetzung nach Anspruch 1, wobei die basischen chemischen Funktionen der organischen Siliciumverbindung aus primären, sekundären oder tertiären Aminen ausgewählt sind.

3. Kosmetische Zusammensetzung nach Anspruch 1 oder 2, wobei die hydrolysierbaren Gruppen aus Alkoxy-, Aryloxy- und Halogengruppen ausgewählt sind.

4. Kosmetische Zusammensetzung nach einem der Ansprüche 1 bis 3, wobei die organische Silizium verbindung bzw. die organischen Siliciumverbindungen aus den Verbindungen der Formel (I) ausgewählt ist bzw. sind: 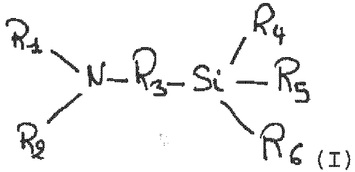 wobei:
R₄ für ein Halogen oder eine Gruppe OR' oder R'₁ steht;
R₅ für ein Halogen oder eine Gruppe OR" oder R'₂ steht;
R₆ für ein Halogen oder eine Gruppe OR''' oder R'₃ steht;
R₁, R₂, R₃, R', R", R"', R'₁, R'₂ und R'₃ unabhängig voneinander für eine lineare oder verzweigte, gesättigte oder ungesättigte Kohlenwasserstoffgruppe, die gegebenenfalls zusätzliche chemische Gruppen trägt, stehen, wobei R₁, R₂, R', R" und R"' außerdem für Wasserstoff stehen können und mindestens zwei der Gruppen R₄, R₅ und R₆ von den Gruppen R'₁, R'₂ und R'₃ verschieden sind.

5. Kosmetische Zusammensetzung nach Anspruch 4, wobei die Gruppen R₁, R₂, R', R'₁, R'₂, R'₃, R" und R"' aus C₁-C₁₂-Alkylresten, C₅-C₁₄-Arylresten, (C₁-C₈)Alkyl(C₅-C₁₄)arylresten und (C₅- C₁₄)Aryl(C₁-C₈)alkylresten ausgewählt sind und die Gruppe R₃ aus C₁-C₁₂-Alkylenresten, die gegebenenfalls durch eine Aminogruppe substituiert sind, C₅-C₁₄-Arylenresten, (C₁-C8)Alkylen(C₅-C₁₄)arylenresten und (C₅-C₁₄)Arylen(C₁-C₈)alkylenresten ausgewählt ist.

6. Kosmetische Zusammensetzung nach einem der Ansprüche 1 bis 3, wobei die organische Siliciumverbindung bzw. die organischen Siliciumverbindungen aus den Verbindungen der Formel (II) ausgewählt ist bzw. sind:
(R₂₁O)ₓ(R₂₂)_{y}Si-(B)ₚ-[NR₂₃-(B')_{p'}-]_{q}- [N R'₂₃-(B")_{p'}]_{q'}-Si-(R'₂₂)_{y'}(OR'₂₁)_{x'} (II)
wobei
R₂₁, R₂₂, R'₂₁ und R'₂₂ jeweils unabhängig für eine lineare oder verzweigte, gesättigte oder ungesättigte Kohlenwasserstoffkette, die gegebenenfalls ein oder mehrere Heteroatome enthält und gegebenenfalls durch eine oder mehrere Gruppen, die aus Ether-, Ester-, Amin-, Amid-, Carboxyl-, Hydroxyl- und Carbonylgruppen ausgewählt sind, unterbrochen oder substituiert ist, stehen,
x für eine ganze Zahl im Bereich von 1 bis 3 steht,
y = 3-x,
x¹ für eine ganze Zahl im Bereich von 1 bis 3 steht,
y' = 3-x'
p = 0 oder 1,
p' = 0 oder 1,
p'' = 0 oder 1,
q = 0 oder 1,
q' = 0 oder 1,
mit der Maßgabe, dass mindestens q oder q' von null verschieden ist,
B, B' und B'' jeweils unabhängig für einen zweiwertigen linearen oder verzweigten C₁₋₂₀-Alkylenrest stehen,
R₂₃ und R'₂₃ jeweils unabhängig für ein Wasserstoffatom oder eine lineare oder verzweigte, gesättigte oder ungesättigte Kohlenwasserstoffkette, die gegebenenfalls ein oder mehrere Heteroatome enthält und gegebenenfalls durch eine oder mehrere Ether-, C₁-C₂₀-Alkoholester-, Amin-, Carboxyl-, Alkoxysilan-, C₆-C₃₀-Aryl-, Hydroxyl- oder Carbonylgruppen unterbrochen oder substituiert ist, oder einen heterocyclischen oder nicht heterocyclischen aromatischen Ring, der gegebenenfalls durch eine oder mehrere C₃-C₂₀-Alkoholester-, Amin-, Amid-, Carboxyl-, Alkoxysilan-, Hydroxyl-, Carbonyl- oder Acylgruppen substituiert ist, stehen.

7. Kosmetische Zusammensetzung nach einem der vorhergehenden Ansprüche, wobei es sich bei der organischen Siliciumverbindung um 3-Aminopropyltriethoxysilan, 3-Aminopropylmethyldiethoxysilan, N-(2-Aminoethyl)-3-aminopropyltriethoxysilan oder 3-(2-Aminoethylamino)propylmethyldiethoxysilan handelt.

8. Kosmetische Zusammensetzung nach einem der vorhergehenden Ansprüche, wobei das hydrophobe filmbildende Polymer bzw. die hydrophoben filmbildenden Polymere aus Polyurethanen; Polyurethanacrylaten; Polyharnstoffen; Polyharnstoff-polyurethanen; Polyesterpolyurethanen; Polyetherpolyurethanen; Polyestern; Polyesteramiden; Acrylpolyestern; Polymeren oder Copolymeren auf Basis von Polyvinylpyrrolidon; Acryl- und/oder Vinylpolymeren oder -copolymeren; Polyacrylamiden; Silikonpolymeren mit Acrylteilen; Silikonharzen; Polyharnstoff/Polyurethan-Silikonen; Copolymeren auf Basis von Silikonharzen und Dimethiconol; Fluorpolymeren; Cellulosen und Mischungen davon ausgewählt ist bzw. sind.

9. Kosmetische Zusammensetzung nach einem der vorhergehenden Ansprüche, wobei das flüchtige Lösungsmittel bzw. die flüchtigen Lösungsmittel aus Wasser und organischen Lösungsmitteln, die aus Ethanol, Isopropanol, Aceton, Isododecan, Decamethylcyclopentasiloxan, Octamethyltrisiloxan und Decamethyltetrasiloxan ausgewählt sind, ausgewählt ist bzw. sind.

10. Kosmetische Zusammensetzung nach einem der vorhergehenden Ansprüche, wobei das Pigment bzw. die Pigmente aus anorganischen Pigmenten, organischen Pigmenten, Lacken, Effektpigmenten wie Perlmutten oder Pailletten und Mischungen davon ausgewählt ist bzw. sind.

11. Kosmetische Zusammensetzung nach Anspruch 10, wobei es sich bei dem Pigment um einen Perlmutt handelt.

12. Verfahren zur kosmetischen Behandlung von Keratinfasern, bei dem man die kosmetische Zusammensetzung gemäß einem der vorhergehenden Ansprüche auf die Fasern aufbringt und die Zusammensetzung bei einer Temperatur von mehr als 40°C trocknet.

13. Verwendung einer kosmetischen Zusammensetzung nach einem der Ansprüche 1 bis 11 zum Färben von Keratinfasern.

14. Verwendung einer kosmetischen Zusammensetzung, die in einem kosmetisch unbedenklichen Medium eine oder mehrere organische Siliciumverbindungen, die aus Silanen mit einem, zwei oder drei Siliciumatomen ausgewählt sind, wobei die organischen Siliciumverbindungen außerdem eine oder mehrere basische chemische Funktionen und eine oder mehrere Hydroxylgruppen oder hydrolysierbare Gruppen pro Molekül umfassen, umfasst, auf Keratinfasern als Vorbehandlung für eine kosmetische Zusammensetzung, die ein oder mehrere hydrophobe filmbildende Polymere, ein oder mehrere Pigmente und ein oder mehrere flüchtige Lösungsmittel enthält.

15. Verfahren zur kosmetischen Behandlung von Keratinfasern, das darin besteht, dass man eine kosmetische Vorbehandlungszusammensetzung, die in einem kosmetisch unbedenklichen Medium eine oder mehrere organische Siliciumverbindungen gemäß einem der Ansprüche 1 bis 7 umfasst, aufbringt und dann eine kosmetische Zusammensetzung, die ein oder mehrere hydrophobe filmbildende Polymere, ein oder mehrere Pigmente und ein oder mehrere flüchtige Lösungsmittel umfasst, aufbringt,

## Claims

1. Cosmetic composition for treating keratin fibres, comprising:
- one or more organosilicon compounds chosen from silanes comprising one, two or three silicon atoms, the said organosilicon compounds also comprising one or more basic chemical functions and one or more hydroxyl or hydrolysable groups per molecule,
- one or more hydrophobic film-forming polymers,
- one or more pigments, and
- one or more volatile solvents.

2. Cosmetic composition according to Claim 1, in which the basic chemical functions of the organosilicon compound are chosen from primary, secondary and tertiary amines.

3. Cosmetic composition according to Claim 1 or 2, in which the hydrolysable groups are chosen from alkoxy, aryloxy and halogen groups.

4. Cosmetic composition according to any one of Claims 1 to 3, in which the organosilicon compound(s) is (are) chosen from the compounds of formula (I): 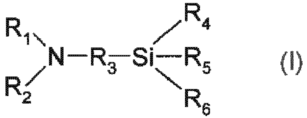 in which:
R₄ represents a halogen or a group OR' or R'₁;
R₅ represents a halogen or a group OR" or R'₂;
R₆ represents a halogen or a group OR''' or R'₃;
R₁, R₂, R₃, R', R" , R"', R'₁, R'₂ and R'₃ represent, independently of each other, a saturated or unsaturated, linear or branched hydrocarbon-based group optionally bearing additional chemical groups, R₁₁, R₂, R', R" and R'" also possibly denoting hydrogen, and at least two of the groups R₄, R₅ and R₆ being other than the groups R'₁, R'₂ and R'₃.

5. Cosmetic composition according to Claim 4, in which the groups R₁, R₂, R', R'₁, R'₂, R'₃, R" and R"' are chosen from C₁-C₁₂ alkyl radicals, C₅-C₁₄ aryl radicals, (C₁-C₈)alkyl(C₅-C₁₄)aryl radicals and (C₅-C₁₄) aryl (C₁-C₈) alkyl radicals and the group R₃ is chosen from C₁-C₁₂ alkylene radicals, optionally substituted with an amino group, C₅-C₁₄ arylene radicals, (C₁-C₈)alkylene(C₅-C₁₄)arylene radicals and (C₅-C₁₄)arylene(C₁-C₈)alkylene radicals.

6. Cosmetic composition according to any one of Claims 1 to 3, in which the organosilicon compound(s) is (are) chosen from the compounds of formula (II)
(R₂₁O)ₓ(R₂₂)_{y}Si-(B)ₚ-[NR₂₃-(B')_{p'}]_{q}-[NR'₂₃-(B")_{p"}]_{q}-Si-(R'₂₂)_{y}-(OR'₂₁)_{x'} (II)
in which:
R₂₁, R₂₂, R'₂₁ and R'₂₂ each independently represent a saturated or unsaturated, linear or branched hydrocarbon-based chain, optionally containing one or more heteroatoms, optionally interrupted or substituted with one or more groups chosen from ether, ester, amine, amide, carboxyl, hydroxyl and carbonyl groups,
x is an integer ranging from 1 to 3,
y = 3-x,
x' is an integer ranging from 1 to 3,
y' = 3-x',
p = 0 or 1,
p' = 0 or 1,
p" = 0 or 1,
q = 0 or 1,
q' = 0 or 1,
it being understood that at least q or q' is other than zero,
B, B' and B" each independently represent a linear or branched divalent C₁-C₂₀ alkylene radical,
R₂₃ and R'₂₃ each independently represent a hydrogen atom or a saturated or unsaturated, linear or branched hydrocarbon-based chain, optionally containing one or more heteroatoms, optionally interrupted or substituted with one or more ether, ester of a C₁-C₂₀ alcohol, amine, carboxyl, alkoxysilane, C₆-C₃₀ aryl, hydroxyl or carbonyl groups, or a heterocyclic or non-heterocyclic aromatic ring, optionally substituted with one or more ester of a C₃-C₂₀ alcohol, amine, amide, carboxyl, alkoxysilane, hydroxyl, carbonyl or acyl groups.

7. Cosmetic composition according to any one of the preceding claims, in which the organosilicon compound is 3-aminopropyltriethoxysilane, 3-aminopropylmethyldiethoxysilane, N-(2-aminoethyl)-3-aminopropyltriethoxysilane or 3-(2-aminoethylamino)-propylmethyldiethoxysilane.

8. Cosmetic composition according to any one of the preceding claims, in which the hydrophobic film-forming polymer(s) is (are) chosen from polyurethanes; polyurethane-acrylics; polyureas; polyurea-polyurethanes; polyester-polyurethanes; polyether-polyurethanes; polyesters; polyester amides; acrylic polyesters; polyvinylpyrrolidone-based polymers or copolymers; acrylic and/or vinyl polymers or copolymers; polyacrylamides; silicone polymers comprising acrylic parts; silicone resins; polyurea/polyurethane silicones; copolymers based on silicone resin and dimethiconol; fluoro polymers; celluloses, and mixtures thereof.

9. Cosmetic composition according to any one of the preceding claims, in which the volatile solvent(s) is (are) chosen from water and organic solvents chosen from ethanol, isopropanol, acetone, isododecane, decamethylcyclopentasiloxane, octamethyltrisiloxane and decamethyltetrasiloxane.

10. Cosmetic composition according to any one of the preceding claims, in which the pigment(s) is (are) chosen from mineral pigments, organic pigments, lakes, and pigments with special effects such as nacres or glitter flakes, and mixtures thereof.

11. Cosmetic composition according to Claim 10, in which the pigment is a nacre.

12. Cosmetic process for treating keratin fibres, in which the cosmetic composition as defined in any one of the preceding claims is applied to the said fibres, and the composition is dried at a temperature above 40°C.

13. Use of a cosmetic composition according to any one of Claims 1 to 11 for dyeing keratin fibres.

14. Use on keratin fibres of a cosmetic composition comprising, in a cosmetically acceptable medium, one or more organosilicon compounds chosen from silanes comprising one, two or three silicon atoms, the said organosilicon compounds also comprising one or more basic chemical functions and one or more hydroxyl or hydrolysable groups per molecule, as a pretreatment to a cosmetic composition containing one or more hydrophobic film-forming polymers, one or more pigments and one or more volatile solvents.

15. Cosmetic process for treating keratin fibres, which consists in applying a cosmetic pretreatment composition comprising, in a cosmetically acceptable medium, one or more organosilicon compounds as defined according to any one of Claims 1 to 7, and then in applying a cosmetic composition comprising one or more hydrophobic film-forming polymers, one or more pigments and one or more volatile solvents.
